# EUROPEAN PATENT SPECIFICATION

(11) **EP 2 086 538 B1**
(45) Date of publication and mention of the grant of the patent: **14.03.2012**
(21) Application number: 07867284.7
(22) Date of filing: 26.10.2007
(51) Int. Cl.: A61K 31/437, A61K 31/445, A61K 45/06, A61P 25/28

(54) **COMBINATION THERAPIES FOR TREATING ALZHEIMER'S DISEASE USING DIMEBON AND DONEPEZIL**
KOMBINATIONSTHERAPIEN ZUR BEHANDLUNG VON MORBUS ALZHEIMER MIT DIMEBON UND DONEPEZIL
COMBINAISON DE THÉRAPIES POUR LE TRAITEMENT DE LA MALADIE D'ALZHEIMER AVEC DIMEBON ET DONEPEZIL

(30) Priority: 27.10.2006 US 854866 P
(43) Date of publication of application: 12.08.2009
(60) Divisional application: 11181674.0
(73) Proprietor: Medivation Neurology, Inc., San Francisco, CA 94105 (US)
(72) Inventor: HUNG, David T., Redwood City, CA 94062 (US); PROTTER, Andrew Asher, Palo Alto, CA 94301 (US)
(74) Representative: Sexton, Jane Helen
(86) International application number: PCT/US2007/022645
(87) International publication number: WO 2008/051599

(56) References cited:
- US-B1- 6 187 785
- LERMONTOVA N N ET AL: "Dimebon and Tacrine Inhibit Neurotoxic Action of [beta]-Amyloid in Culture and Block L-type Ca2+ Channels" BULLETIN OF EXPERIMENTAL BIOLOGY AND MEDICINE, KLUWER ACADEMIC PUBLISHERS-PLENUM PUBLISHERS, NE, vol. 132, no. 5, 1 November 2001 (2001-11-01), pages 1079-1083, XP019217726 ISSN: 1573-8221
- SCARPINI ET AL: "Treatment of Alzheimer's disease; current status and new perspectives" LANCET NEUROLOGY, LANCET PUBLISHING GROUP, LONDON, GB, vol. 2, no. 9, 1 September 2003 (2003-09-01), pages 539-547, XP005108654 ISSN: 1474-4422
- HARTMANN S ET AL: "Tolerability of memantine in combination with cholinesterase inhibitors in dementia therapy" INTERNATIONAL CLINICAL PSYCHOPHARMACOLOGY, CLINICAL NEUROSCIENCE PUBLISHERS, LONDON, GB, vol. 18, no. 2, 1 January 2003 (2003-01-01), pages 81-85, XP002967315 ISSN: 0268-1315
- BACHURIN S ET AL: "Antihistamine agent Dimebon as a novel neuroprotector and a cognition enhancer." ANNALS OF THE NEW YORK ACADEMY OF SCIENCES JUN 2001, vol. 939, June 2001 (2001-06), pages 425-435, XP002478538 ISSN: 0077-8923
- GROSSBERG G T ET AL: "Current strategies for the treatment and prevention of Alzheimer's disease" PRIMARY PSYCHIATRY, MBL COMMUNICATIONS, NEW YORK, NY, US, vol. 14, no. 8, 2007, pages 39-54, XP009099265 ISSN: 1082-6319

## Description

### TECHNICAL FIELD

The present invention relates to a compound which is 2,8-dimethyl-5-(2-(6-methyl-3-pyridyl)ethyl)-2,3,4,5-tetrahydro-1H-pyrido[4,3-b]indole or a pharmaceutically acceptable salt thereof useful in a method for treating, preventing and/or delaying the onset and/or development of Alzheimer's disease by administering to an individual a first therapy comprising said compound and a second therapy comprising donepezil, or a pharmaceutically acceptable salt thereof.

### BACKGROUND OF THE INVENTION

### Summary of Alzheimer's Disease Pathology

Alzheimer's disease is currently one of the severest and most widely spread neurodegenerative diseases. Alzheimer's disease is a degenerative brain disorder characterized clinically by progressive memory deficits, confusion, behavioral problems, inability to care for oneself, gradual physical deterioration and, ultimately, death. Approximately 15 million people worldwide are affected by Alzheimer's disease, and the number is expected to increase dramatically as lifespans increase. Histologically, the disease is characterized by neuritic plaques, found primarily in the association cortex, limbic system and basal ganglia. The major constituent of these plaques is amyloid beta peptide (Aβ), which is the cleavage product of beta amyloid precursor protein (βAPP or APP). APP is a type I transmembrane glycoprotein that contains a large ectopic N-terminal domain, a transmembrane domain and a small cytoplasmic C-terminal tail. Alternative splicing of the transcript of the single APP gene on chromosome 21 results in several isoforms that differ in the number of amino acids.

Aβ appears to have a central role in the neuropathology of Alzheimer's disease. Familial forms of the disease have been linked to mutations in APP and the presenilin genes (Tanzi et al., 1996, Neurobiol. Dis., 3:159-168; Hardy, 1996, Ann. Med., 28:255-258). Diseased-linked mutations in these genes result in increased production of the 42-amino acid form of Aβ, the predominant form found in amyloid plaques.

Currently, there is no cure for Alzheimer's disease. Presently available therapies are directed to treating symptoms associated with Alzheimer's disease.

### Summary of Hydrogenated Pyrido [4,3-b] Indole Derivatives

Known compounds of the class of tetra- and hexahydro-1H-pyrido[4,3-b]indole derivatives manifest a broad spectrum of biological activity. In the series of 2,3,4,5-tetrahydro-1H-pyrido[4,3-b]indoles the following types of activity have been found: antihistamine activity (DE 1,813,229, filed Dec. 6, 1968; DE 1,952,800, filed Oct. 20, 1969), central depressive and anti-inflammatory activity (U.S. Pat. No. 3,718,657, filed Dec. 3, 1970), neuroleptic activity (Herbert C. A., Plattner S. S., Welch W. M., Mol. Pharm. 1980, Vo1.17, No. 1, p.38-42) and others. 2,3,4,4a,5,9b-hexahydro-1H-pyrido[4,3-b]indole derivatives show psychotropic (Welch W. M., Harbert C. A., Weissman A., Koe B. K., J.Med.Chem.,1986, Vol.29, No. 10, p.2093-2099), antiaggressive, antiarrhythmic and other types of activity.

Several drugs, such as diazoline (mebhydroline), dimebon, dorastine, carbidine (dicarbine), stobadine and gevotroline, based on tetra- or hexahydro-1H-pyrido[4,3-b]indole derivatives are known to have been manufactured. Diazoline (2-methyl-5-benzyl-2,3,4,5-tetrahydro-1H-pyrido[4,3-b]indole dihydrochloride) (Klyuev M. A., Drugs, used in "Medical Pract.", USSR, Moscow, "Meditzina" Publishers, 1991, p.512) and dimebon (2,8-dimethyl-5-(2-(6-methyl-3-pyridyl)ethyl-2,3,4,5-tetrahydro-1H-pyrido[4,3-b]indole dihydrochloride) (M. D. Mashkovsky, "Medicinal Drugs" in Vol. 1, 12th Edition, Moscow, "Meditzina" Publishers, 1993, p.383) as well as dorastine (2-methyl-8-chloro-5-[2-(6-methyl-3-pyridyl)ethyl]-2,3,4,5-tetrahydro-1H-pyrido[4,3-b]indole dihydrochloride) (USAN and USP dictionary of drugs names (United States Adopted Names, 1961-1988, current US Pharmacopoeia and National Formula for Drugs and other nonproprietary drug names), 1989, 26th Edition., p.196) are known as antihistamine drugs; carbidine (dicarbine) (is(±)-2,8-dimethyl-2,3,4,4a,5,9b-hexahydro-1H-pyrido[4,3-b]indole dihydrochloride) is a neuroleptic agent having an antidepressive effect (L. N. Yakhontov, R. G. Glushkov, Synthetic Drugs, ed. by A. G. Natradze, Moscow, "Meditzina" Publishers, 1983, p.234-237), and its (-)isomer, stobadine, is known as an antiarrythmic agent (Kitlova M., Gibela P., Drimal J., Bratisl. Lek.Listy, 1985, Vol.84, No.5, p.542-549); gevotroline 8-fluoro-2-(3-(3-pyridyl)propyl)-2,3,4,5-tetrahydro-1H-pyrido[4,3-b]indole dihydrochloride is an antipsychotic and anxiolytic agent (Abou - Gharbi M., Patel U. R., Webb M. B., Moyer J. A., Ardnee T. H., J. Med. Chem., 1987, vol.30, p.1818-1823). Dimebon has been used in medicine as an antiallergic agent (Inventor's Certificate No. 1138164, IP Class A61K 31/47,5, C07 D 209/52, published on Feb. 7, 1985) in Russia for over 20 years.

As described in U.S. Patent No. 6,187,785, hydrogenated pyrido[4,3-b]indole derivatives, such as dimebon, have NMDA antagonist properties, which make them useful for treating neurodegenerative diseases, such as Alzheimer's disease. See also U.S. Patent No. 7,071,206. As described in WO 2005/055951, hydrogenated pyrido[4,3-b]indole derivatives, such as dimebon, are useful as human or veterinary geroprotectors *e.g*., by delaying the onset and/or development of an age-associated or related manifestation and/or pathology or condition, including disturbance in skin-hair integument, vision disturbance and weight loss. U.S. Provisional Patent Application No. 60/723,403, filed October 4, 2006, and U.S. Patent Application No. 11/543,529, filed October 4, 2006, disclose hydrogenated pyrido[4,3-b]indole derivatives, such as dimebon, as neuroprotectors for use in treating and/or preventing and/or slowing the progression or onset and/or development of Huntington's disease. See also RU application filed January 25, 2006 with an English language title of "Agent for Treatment of Schizophrenia Based on Hydrogenated Pyrido[4,3-b]indoles (Variations), a Pharmacological Agent Based on it, and a Method of Using it."

### Significant Medical Need

There remains a significant interest in and need for additional or alternative therapies for treating, preventing and/or delaying the onset and/or development of Alzheimer's disease. Preferably, the therapeutic agents can alter the underlying disease process and/or course, or improve the quality of life and/or prolong the survival time for patients with Alzheimer's disease.

### BRIEF SUMMARY OF THE INVENTION

Methods, combination therapies, pharmaceutical compositions and kits for treating and/or preventing and/or delaying the onset and/or the development of Alzheimer's disease using a hydrogenated [4,3-b] indole or pharmaceutically acceptable salt thereof and another compound, pharmaceutically acceptable salt thereof or therapy for Alzheimer's disease are described. The methods, combination therapies, pharmaceutical compositions and kits may comprise the compounds detailed herein, including without limitation the compound dimebon (2,8-dimethyl-5-(2-(6-methyl-3-pyridyl)ethyl)-2,3,4,5-tetrahydro-1H-pyrido[4,3-b]indole dihydrochloride) in conjunction with one or more other compounds or therapies useful for treating, preventing, slowing the progression or delaying the onset and/or development of Alzheimer's disease. A neuroprotective drug such as dimebon, *e.g*., a drug that has a beneficial effect on saving the viability of neurons, is believed to be highly advantageous for use in combination therapy with other therapeutic agents, *e.g*., when used in connection with therapies that either are lesser or not neuroprotective or act by a different biochemical mechanism, for the treatment of Alzheimer's disease.
The present invention provides a compound which is 2,8-dimethyl-5-(2-(6-methyl-3-pyridyl)ethyl)-2,3,4,5-tetrahydro-1H-pyrido[4,3-b]indole, or a pharmaceutically acceptable salt thereof for use in a method of treating, preventing, slowing the progression or delaying the onset and/or development of Alzheimer's disease in an individual in need thereof, the method comprising administering to the individual an effective amount of a combination of (i) a first therapy comprising said compound; and (ii) a second therapy comprising donepezil or pharmaceutically acceptable salt thereof.
The compound may be for use in combination with the second therapy in a method of treating Alzheimer's disease in an individual in need thereof; a method of preventing or slowing the onset and/or development of Alzheimer's disease in an individual who has a mutated or abnormal gene associated with Alzheimer's disease (*e.g*., an APP mutation, a presenilin mutation and/or an ApoE4 allele); a method of slowing the progression of Alzheimer's disease in an individual who has been diagnosed with Alzheimer's disease; or a method of preventing or slowing the onset and/or development of Alzheimer's disease in an individual who is at risk of developing Alzheimer's disease (*e.g*., an individual with an APP mutation, a presenilin mutation and/or an ApoE4 allele).

In various embodiments, the first therapy is dimebon. The second therapy includes a compound that increases the amount or activity of acetylcholine: an acetylcholinesterase inhibitor, donepezil (2-[(1-benzyl-4-piperidyl)methyl]-5,6-dimethoxy-2,3-dihydroinden-1-one) or a pharmaceutically acceptable salt thereof, such as donepezil hydrocholoride marketed under the name Aricept^{®} (as used herein, Aricept^{®} intends the chemical entity donepezil such as donepezil hydrochloride and is not limited to compounds marketed under the name Aricept^{®});
The present invention also provides use of a compound which is 2,8-dimethyl-5-(2-(6-methyl-3-pyridyl)ethyl)-2,3,4,5-tetrahydro-1H-pyrido[4,3-b]indole or a pharmaceutically acceptable salt thereof in the manufacture of a medicament for use in treating, preventing, slowing the progression or delaying the onset and/or development of Alzheimer's disease in an individual in need thereof in a method comprising administering to the individual an effective amount of a combination of (i) a first therapy comprising said compound; and (ii) a second therapy comprising donepezil or pharmaceutically acceptable salt thereof.

In another aspect, the invention provides a pharmaceutical composition comprising:
(a) a first therapy comprising 2,8-dimethyl-5-(2-(6-methyl-3-pyridyl)ethyl)-2,3,4,5-tetrahydro-1H-pyrido[4,3-b]indole or pharmaceutically acceptable salt thereof;
(b) a second therapy comprising donepezil or pharmaceutically acceptable salt thereof; and
(c) a pharmaceutically acceptable carrier.
In various embodiments, the first therapy is dimebon; the second therapy is the acetylcholinesterase inhibitor Aricept. In some embodiments, the amount of the first therapy, the second therapy or the combined therapy is an amount sufficient to increase the amount or activity of acetylcholine, reduce an activity of an acetylcholinesterase or reduce an activity of an NMDA receptor. In some embodiments, one or more of these activities changes by at least or about 10%, 20%, 30%, 40%, 50%, 60%, 70%, 80%, 90%, 95% or 100% as compared to the corresponding symptom in the same subject prior to treatment or compared to the corresponding symptom in other subjects not receiving the combination therapy. In some embodiments, the amount of the first therapy, the second therapy or the combined therapy is an amount sufficient to produce a desired therapeutic outcome (*e.g*., reducing the severity and/or duration of, stabilizing the severity of, or eliminating one or more symptoms of Alzheimer's disease). In various embodiments, the amount of the first therapy, the second therapy, or the combined therapy is a an amount sufficient to prevent or reduce the severity and/or onset of one or more future symptoms of Alzheimer's disease when administered to an individual who is susceptible and/or who may develop Alzheimer's disease.

In yet another aspect, the invention includes a product comprising: (a) a first therapy comprising 2,8-dimethyl-5-(2-(6-methyl-3-pyridyl)ethyl)-2,3,4,5-tetrahydro-1H-pyrido[4,3-b]indole, or pharmaceutically acceptable salt thereof, and (b) a second therapy comprising donepezil or pharmaceutically acceptable salt thereof;
as a combined preparation for simultaneous, separate or sequential use in the treatment, prevention, slowing the progression or delaying the onset and/or development of Alzheimer's disease.
In various embodiments, the first therapy is dimebon; the second therapy is the acetylcholinesterase inhibitor Aricept. In some embodiments, the amount of the first therapy, the second therapy or the combined therapy is an amount sufficient to increase the amount or activity of acetylcholine, reduce an activity of an acetylcholinesterase or reduce an activity of an NMDA receptor.
In some embodiments, one or more of these activities changes by at least or about 10%, 20%, 30%, 40%, 50%, 60%, 70%, 80%, 90%, 95% or 100% as compared to the corresponding symptom in the same subject prior to treatment or compared to the corresponding symptom in other subjects not receiving the combination therapy. In some embodiments, the amount of the first therapy, the second therapy or the combined therapy is an amount sufficient to produce a desired therapeutic outcome (*e.g*., reducing the severity or duration of, stabilizing the severity of, or eliminating one or more symptoms of Alzheimer's disease). In various embodiments, the amount of the first therapy, the second therapy, or the combined therapy is a an amount sufficient to prevent or reduce the severity of one or more future symptoms of Alzheimer's disease when administered to an individual who is susceptible and/or who may develop Alzheimer's disease.

### DETAILED DESCRIPTION OF THE INVENTION

### Definitions

Unless clearly indicated otherwise, use of the terms "a", "an" and the like refers to one or more.

Unless clearly indicated otherwise, "an individual" as used herein intends a mammal, including but not limited to a human. The individual may be a human who has been diagnosed with or is suspected of having Alzheimer's disease. The individual may be a human who exhibits one or more symptoms associated with Alzheimer's disease. The individual may be a human who has a mutated or abnormal gene associated with Alzheimer's disease but who has not been diagnosed with Alzheimer's disease. The individual may be a human who is genetically or otherwise predisposed to developing Alzheimer's disease. In one variation, the individual is a human who has not been diagnosed with and/or is not considered at risk for developing Huntington's disease or schizophrenia. In one variation, the individual is a human who does not have a cognition impairment associated with aging or does not have a non-life threatening condition associated with the aging process (such as loss of sight (cataract), deterioration of the dermatohairy integument (alopecia) or an age-associated decrease in weight due to the death of muscular and fatty cells) or a combination thereof.

As used herein, "treatment" or "treating" is an approach for obtaining beneficial or desired results including clinical results. For purposes of this invention, beneficial or desired clinical results include, but are not limited to, one or more of the following: inhibiting or suppressing the formation of amyloid plaques, reducing, removing, or clearing amyloid plaques, improving cognition or reversing cognitive decline, sequestering soluble Aβ peptide circulating in biological fluids, reducing Aβ peptide (including soluble and deposited) in a tissue (*e.g*., the brain), inhibiting and/or reducing accumulation of Aβ peptide in the brain, inhibiting and/or reducing toxic effects of Aβ peptide in a tissue (*e.g*., the brain), decreasing brain atrophy, decreasing one or more symptoms resulting from the disease (*e.g*., abnormalities of memory, problem solving, language, calculation, visuospatial perception, judgment and/or behavior, inability to care for oneself), increasing the quality of life, decreasing the dose of one or more other medications required to treat the disease, delaying the progression of the disease, altering the underlying disease process and/or course, and/or prolonging survival. In some embodiments, the combination therapy reduces the severity of one or more symptoms associated with Alzheimer's disease by at least 10, 20, 30, 40, 50, 60, 70, 80, 90, or 95% compared to the corresponding symptom in the same subject prior to treatment or compared to the corresponding symptom in other subjects not receiving the combination therapy.

As used herein, "delaying" development of Alzheimer's disease means to defer, hinder, slow, retard, stabilize and/or postpone development of the disease and/or slowing the progression or altering the underlying disease process and/or course once it has developed. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of Alzheimer's disease is a method that reduces probability of disease development in a given time frame and/or reduces extent of the disease in a given time frame, when compared to not using the method, including stabilizing one or more symptoms resulting from the disease (*e.g*., abnormalities of memory, problem solving, language, calculation, visuospatial perception, judgment and/or behavior, inability to care for oneself). Such comparisons are typically based on clinical studies, using a statistically significant number of subjects. Alzheimer's disease development can be detectable using standard clinical techniques, such as standard neurological examination, patient interview, neuroimaging, detecting alterations of levels of specific proteins in the serum or cerebrospinal fluid (*e.g*., amyloid peptides and Tau), computerized tomography (CT) or magnetic resonance imaging (MRI). Development may also refer to disease progression that may be initially undetectable and includes occurrence, recurrence and onset.

As used herein, an "at risk" individual is an individual who is at risk of development of Alzheimer's disease. An individual "at risk" may or may not have detectable disease, and may or may not have displayed detectable disease prior to the treatment methods described herein. "At risk" denotes that an individual has one or more so-called risk factors, which are measurable parameters that correlate with development of Alzheimer's disease. An individual having one or more of these risk factors has a higher probability of developing Alzheimer's disease than an individual without these risk factor(s). These risk factors include, but are not limited to, age, sex, race, diet, history of previous disease, presence of precursor disease, genetic (*i.e*., hereditary) considerations, and environmental exposure. Individuals at risk for Alzheimer's disease include, *e.g*., those having relatives who have experienced the disease, those whose risk is determined by analysis of genetic or biochemical markers, those with positive results in a blood test for any signaling proteins present in blood plasma and/or cerebrospinal fluid ("CSF") known to predict clinical Alzheimer's diagnosis (*see, e.g.,* S. Ray et al., "Classification and prediction of clinical Alzheimer's diagnosis based on plasma signaling proteins," Nature Medicine, published online October 14, 2007), and individuals experiencing a loss of sense of smell.. Genetic markers of risk for Alzheimer's disease include mutations in the APP gene, particularly mutations at position 717 and positions 670 and 671 referred to as the Hardy and Swedish mutations, respectively (Hardy, Trends Neurosci., 20:154-9, 1997). Other markers of risk are mutations in the presenilin genes (*e.g.,* PS1 or PS2), ApoE4 alleles, family history of Alzheimer's disease, hypercholesterolemia and/or atherosclerosis.

As used herein, a "compound that increases the amount or activity of acetylcholine" is meant a compound that increases the level or activity of acetylcholine, such as an acetylcholinesterase inhibitor, an acetylcholine receptor agonist, or a compound that promotes the release of acetylcholine. In some embodiments, the compound increases the level or activity of acetylcholine by at least or about 10%, 20%, 30%, 40%, 50%, 60%, 70%, 80%, 90%, 100%, 150%, 200%, 300%, 400%, 500% or more as compared to the corresponding level or activity in the same subject prior to treatment or compared to the corresponding level or activity in other subjects not receiving the combination therapy.

As used herein, an "acetylcholinesterase inhibitor" is a compound that reduces or eliminates an activity of an acetylcholinesterase ("AChE"), such as the hydrolysis of the neurotransmitter acetylcholine into choline and acetic acid. The hydrolysis of acetylcholine is necessary to allow a cholinergic neuron to return to its resting state after activation. In some embodiments, the AChE inhibitor reduces an activity of an acetylcholinesterase by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. Aricept^{®} (donepezil) is an AChE inhibitor.

As used herein, a "butyrylcholinesterase inhibitor" is a compound that reduces or eliminates an activity of a butyrylcholinesterase ("BChE"), such as the hydrolysis of the neurotransmitter acetylcholine into choline and acetic acid. The hydrolysis of acetylcholine is necessary to allow a cholinergic neuron to return to its resting state after activation. In certain embodiments, the BChE inhibitor reduces an activity of a butyrylcholinesterase by at least or about 10%, 20%, 30%, 40%, 50%, 60%, 70%, 80%, 90%, 95%, or 100% as compared to the corresponding activity in the same subject prior to treatment or compared to the corresponding activity in other subjects not receiving the combination therapy. Exemplary BChE inhibitors include Exelon^{®} (rivastigmine tartrate) and cymserine analogs, such as (-)-*N*¹-phenethylnorcymserine (PEC) and (-)-*N*¹,*N*⁸-bisnorcymserine (BNC).

As used herein, an "acetylcholine receptor agonist" is a compound that increases an activity of an acetylcholine receptor, such as a neuronal nicotinic acetylcholine receptor. In some embodiments, the activator increases an activity of an acetylcholine receptor (*e.g*., a neuronal nicotinic acetylcholine receptor) by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. An exemplary compound is TC-1734 (Targacept), which is an orally active neuronal nicotinic acetylcholine receptor agonist with antidepressant, neuroprotective and long-lasting cognitive effects. Microdialysis studies indicate that TC-1734 enhances the release of acetylcholine from the cortex.

As used herein, a "NMDA receptor antagonist" is a compound that reduces or eliminates an activity of an N-methyl-D-aspartate (NMDA) receptor, which is an ionotropic receptor for glutamate. NMDA receptors bind both glutamate and the co-agonist glycine. Thus, an NMDA receptor antagonist can inhibit the ability of glutamate and/or glycine to activate an NMDA receptor. In some embodiments, the NMDA receptor antagonist binds to the active site of an NDMA receptor (*e.g*., a binding site for glutamate and/or glycine) or binds to an allosteric site on the receptor. The interaction between the NMDA receptor antagonist and the NMDA receptor may be reversible or irreversible. In some embodiments, the antagonist reduces an activity of an NMDA receptor by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. Exemplary NMDA receptor antagonists include Memantine (Namenda® sold by Forest, Axura® sold by Merz, Akatinol® sold by Merz, Ebixa® sold by Lundbeck), Neramexane (Forest Labs), Amantadine, AP5 (2-amino-5-phosphonopentanoate, APV), Dextrorphan, Ketamine, MK-801 (dizocilpine), Phencyclidine, Riluzole and 7-chlorokynurenate. The structure of Neramexane is distinct from that of Namenda but they are pharmacologically equivalent.

As used herein, an "inhibitor of amyloid Aβ peptide or amyloid plaque" is a compound that reduces or eliminates the formation of β-amyloid precursor protein (β-APP), reduces or eliminates the formation of Aβ42, reduces or eliminates an activity of amyloid Aβ peptide or amyloid plaque, reduces or eliminates an interaction of a metal with a protein involved in Aβ oligomer formation, reduces or eliminates the formation of amyloid plaques (*e.g*., amyloid plaques in the brain), increases or promotes the clearance of amyloid plaques (*e.g*., amyloid plaques in the brain), binds to amyloid plaques (*e.g*., amyloid plaques in the brain), stimulates or increases an immune response against β-amyloid peptide, reduces or eliminates the amyloid cascade and/or reduces or eliminates Aβ peptide-induced toxicity or cell death. In some embodiments, the compound reduces the amount of β-APP, Aβ42, amyloid plaque (*e.g*., amyloid plaque in the brain), Aβ peptide-induced toxicity or cell death by at least or about 10%, 20%, 30%, 40%, 50%, 60%, 70%, 80%, 90%, 95% or 100% as compared to the corresponding amount in the same subject prior to treatment or compared to the corresponding amount in other subjects not receiving the combination therapy. Exemplary compounds include 3-amino-1-propanesulfonic acid (Tramiprosate, Alzhemed™) by Neurochem (Gervais et al., "Targeting soluble Abeta peptide with Tramiprosate for the treatment of brain amyloidosis," Neurobiol Aging, May 1, 2006), Posiphen™ (Axonyx), Flurizan (Myriad), Kiacta or Fibrillex (NC-503, Eprodisate disodium, sodium 1,3-propanedisulfonate, 1,3-propanedisulphonic acid, 1,3-PDS), PBT-2 (Prana), Memryte (leuprolide) (Voyager), AN-1792 (Elan/Wyeth), AAB-001 (Elan/Wyeth), and ACC-001 (Elan/Wyeth). The mechanisms of actions of these compounds are described further below.

As used herein, by "cGMP-specific phosphodiesterase type 5 (PDE5) inhibitor" is meant a compound that reduces or inhibits an activity of a cGMP-specific phosphodiesterase type 5 (PDE5), such as the hydrolysis of cGMP. In some embodiments, the PDE5 inhibitor reduces the activity of PDE5 by at least or about 2, 5, 10, 100, 500, 1000, 2000, 3000, 40000-fold or more than it reduces the activity of PDE3, which is involved in control of cardiac contractibility, or the activity of PDE6, which is involved in the phototransduction pathway of the retina. In some embodiments, the inhibitor reduces an activity of PDE5 by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. Exemplary PDE5 inhibitors are 1-[[3-(6,7-dihydro-1-methyl-7-oxo-3-propyl-1*H*pyrazolo[4,3-d]pyrimidin-5-yl)-4-ethoxyphenyl]sulfonyl]-4-methylpiperazine citrate (silednafil, Viagra, sold by Pfizer), (6R-trans)-6-(1,3-benzodioxol-5-yl)-2,3,6,7,12,12a-hexahydro-2-methyl-pyrazino [1', 2':1,6] pyrido[3,4-b]indole-1,4-dione (tadalafil, Cialis, sold by LillyICOS), Levitra (vardenafil, sold by Bayer Pharmaceutical and Glaxo-Smith-Kline-Beecham/ Schering Plough) and zaprinast (Nakamizo et al., "Phosphodiesterase inhibitors are neuroprotective to cultured spinal motor neurons," J Neurosci. Res., 71(4):485-95, Feb, 15, 2003). Viagra is approximately 4,000-fold more effective against PDE5 than against PDE3, and 10-fold more effective against PDE5 than against PDE6.

As used herein, by "monoamine oxidase inhibitor" is meant a compound that reduces or eliminates an activity of a monoamine oxidase, such as the deamination of a monoamine neurotransmitter. These inhibitors preferably reduce or prevent the breakdown of monoamine neurotransmitters. There are two isoforms of monoamine oxidase, MAO-A and MAO-B. MAO-A preferentially deaminates serotonin, melatonin, adrenaline and noradrenaline. MAO-B preferentially deaminates phenylethylamine and trace amines. Dopamine is equally deaminated by both types. In some embodiments, the inhibitor reduces an activity of a monoamine oxidase by at or about least 10%, 20%, 30%, 40%, 50%, 60%, 70%, 80%, 90%, 95%, or 100% as compared to the corresponding activity in the same subject prior to treatment or compared to the corresponding activity in other subjects not receiving the combination therapy. An exemplary monoamine oxidase inhibitor is 5-(N-methyl-N-propargyaminomethyl)-8-hydroxyquinoline, also referred to as M30. This compound is a multifunctional MAO-B inhibitor, neuroprotective, iron chelator (Zheng et al., "Novel multifunctional neuroprotective iron chelator-monoamine oxidase inhibitor drugs for neurodegenerative diseases: in vitro studies on antioxidant activity, prevention of lipid peroxide formation and monoamine oxidase inhibition. J Neurochem., 2005 Oct;95(1):68-78). Other exemplary monoamine oxidase inhibitors include isocarboxazid (Marplan), moclobemide (Aurorix, Manerix, Moclodura®), phenelzine (Nardil), tranylcypromine (Parnate), selegiline (Selegiline, Eldepryl), emsam, nialamide, iproniazid (Marsilid, Iprozid, Ipronid, Rivivol, Propilniazida), iproclozide, ladostigil and toloxatone. Many tryptamines, such as harmine, AMT, 5-MeO-DMT and 5-MeO-AMT, have monoamine oxidase inhibitors properties.

As used herein, by "vascular endothelial cell growth factor (VEGF)" is meant a VEGF protein or fragment thereof, such as any protein that results from alternate splicing of mRNA from a single, 8 exon, VEGF gene or homolog thereof. The different VEGF splice variants are referred to by the number of amino acids they contain. In humans, the isoforms are VEGF121, VEGF145, VEGF165, VEGF189 and VEGF206; the rodent orthologs of these proteins contain one less amino acid. These proteins differ by the presence or absence of short C-terminal domains encoded by exons 6a, 6b and 7 of the VEGF gene. These domains have important functional consequences for the VEGF splice variants as they mediate interactions with heparan sulfate proteoglycans and neuropilin co-receptors on the cell surface, enhancing their ability to bind and activate the VEGF signaling receptors. VEGF exerts neuroprotective effects via its cell surface receptor Flk-1. Flk-1 activates PI3 kinase/AKT and ERK to exert a neuroprotective effect (Matsuzaki et al., "Vascular endothelial growth factor rescues hippocampal neurons from glutamate-induced toxicity: signal transduction cascades," FASEB J., 2001 May; 15(7): 1218-20). In various embodiments, the amino acid sequence of the VEGF protein or protein fragment is at least or about 50%, 60%, 70%, 80%, 90%, 95% or 100% identical to that of the corresponding region of a human VEGF protein. In some embodiments, the VEGF fragment contains at least 25, 50, 75, 100, 150 or 200 contiguous amino acids from a full-length VEGF protein and has at least or about 5%, 10%, 20%, 30%, 40%, 50%, 60%, 70%, 80%, 90% or 100% of an activity of a corresponding full-length VEGF protein.

As used herein, by "trophic growth factor" is meant a compound that stimulates cellular proliferation, cellular differentiation, and/or cell survival. Exemplary trophic growth factors include IGF-1, FGF, NGF, BDNF, GCS-F, GMCS-F, mimics and fragments thereof. In various embodiments, the amino acid sequence of a trophic growth factor or fragment thereof is at least 50%, 60%, 70%, 80%, 90%, 95% or 100% identical to that of the corresponding region of a human growth factor. In some embodiments, the growth factor fragment contains at least 25, 50, 75, 100, 150 or 200 contiguous amino acids from a full-length growth factor and has at least or about 5%, 10%, 20%, 30%, 40%, 50%, 60%, 70%, 80%, 90% or 100% of an activity of a corresponding full-length growth factor.

As used herein, by "hypoxia inducible factor (HIF) activator" is meant a compound that increases an activity of a HIF. HIFs are transcription factors that respond to changes in available oxygen in the cellular environment, such as decreases in oxygen or hypoxia. In some embodiments, the activator increases an activity of a HIF by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy.

As used herein, by "HIF prolyl 4-hydroxylase inhibitor' is meant a compound that reduces or eliminates an activity of a HIF prolyl 4-hydroxylase. The alpha subunit of HIF-1 is a target for prolyl hydroxylation by HIF prolyl-hydroxylase, which makes HIF-1 alpha a target for degradation by the E3 ubiquitin ligase complex. In some embodiments, the inhibitor reduces an activity of a HIF prolyl 4-hydroxylase by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy.

As used herein, by "anti-apoptotic compound" is meant a compound that reduces or eliminates programmed cell death. In some embodiments, the compound reduces cell death (*e.g*., neuronal cell death in the brain or a region of the brain) by at least or about 10%, 20%, 30%, 40%, 50%, 60%, 70%, 80%, 90%, 95% or 100% as compared to the corresponding cell death in the same subject prior to treatment or compared to the corresponding cell death in other subjects not receiving the combination therapy.

As used herein, by "activity-dependent neuroprotective protein (ADNP) agonist or analog" is meant a compound that increases or mimics an activity of an ADNP. ADNP has been identified as a glial derived protein that has neuroprotective activity. In some embodiments, the compound increases an activity of an ADNP by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. In various embodiments, the amino acid sequence of the ADNP agonist or analog is at least or about 50%, 60%, 70%, 80%, 90%, 95% or 100% identical to that of the corresponding region of a human ADNP. In some embodiments, the ADNP agonist or analog contains at least or about 5, 10, 25, 50, 75, 100, 150 or 200 contiguous amino acids from a full-length ADNP and has at least or about 5%, 10%, 20%, 30%, 40%, 50%, 60%, 70%, 80%, 90% or 100% of an activity of a corresponding full-length ADNP. An exemplary ADNP analog is AL-108 (Allon), which is an intranasally formulated eight amino acid neuroprotective peptide analog of ADNP.

As used herein, by "activity-dependent neurotrophic factor (ADNF) agonist or analog" is meant a compound that increases or mimics an activity of an ADNF. ADNF has been identified as a glial derived factor that has neuroprotective activity. In some embodiments, the compound increases an activity of an ADNF by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. In various embodiments, the amino acid sequence of the ADNF agonist or analog is at least or about 50%, 60%, 70%, 80%, 90%, 95% or 100% identical to that of the corresponding region of a human ADNF. In some embodiments, the ADNF agonist or analog contains at least or about 5, 10, 25, 40, or 50 contiguous amino acids from a full-length ADNF and has at least or about 5%, 10%, 20%, 30%, 40%, 50%, 60%, 70%, 80%, 90% or 100% of an activity of a corresponding full-length ADNF. An exemplary ADNF peptide agonist is AL-208 (Allon), which is a nine amino acid peptide, Ser-Ala-Leu-Leu-Arg-Ser-Ile-Pro-Ala (SALLRSIPA), with neuroprotective activity.

As used herein, by "activator or positive modulator of an AMPA-type glutamate receptor" is meant a compound that increases an activity of an AMPA-type glutamate receptor. In some embodiments, the activator increases an activity of an AMPA-type glutamate receptor by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. CX717 (Cortex) and CX516 (Cortex) are exemplary positive modulators of the AMPA-type glutamate receptor.

As used herein, by "serotonin 5-HT1A receptor agonist" is meant a compound that increases an activity of serotonin 5-HT1A receptor. In some embodiments, the compound increases an activity of a serotonin 5-HT1A receptor by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. Xaliproden (Sanofi-Aventis) [SR 57746A, xaliprodene; Xaprila] is an exemplary serotonin 5-HT1A receptor agonist that also mimics the effects of nerve growth factor.

As used herein, by "serotonin 1A receptor antagonist' is meant a compound that reduces or eliminates an activity of a serotonin 1A receptor. In some embodiments, the inhibitor reduces an activity of a serotonin 1A receptor by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. In some embodiments, the serotonin 1A receptor antagonist binds to the active site of a serotonin 1A receptor (*e.g*., a binding site for a ligand) or binds to an allosteric site on the receptor. The interaction between the serotonin 1A receptor antagonist and the serotonin 1A receptor may be reversible or irreversible. Lecozotan (SRA-333, Wyeth) is an exemplary selective serotonin 1A receptor antagonist that enhances the stimulated release of glutamate and acetylcholine in the hippocampus and possesses cognitive-enhancing properties.

As used herein, by "nicotinic alpha-7 receptor agonist" is meant a compound that increases an activity of a nicotinic alpha-7 receptor. In some embodiments, the compound increases an activity of a nicotinic alpha-7 receptor by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. An exemplary compound includes MEM 3454 (Memory Pharma), which is a partial agonist of the nicotinic alpha-7 receptor.

As used herein, by "neuronal L-type calcium channel modulator" is meant a compound that increases or decreases an activity of a neuronal L-type calcium channel. In some embodiments, the compound alters an activity of a neuronal L-type calcium channel by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. An exemplary compound includes MEM 1003 (Memory Pharma).

As used herein, by "5-HT4 receptor agonist" is meant a compound that increases an activity of a 5-HT4 receptor. In some embodiments, the compound increases an activity of a 5-HT4 receptor by at least or about 10%, 20%, 30%, 40%, 50%, 60%, 70%, 80%, 90%, 100%, 150%, 200%, 300%, 400%, 500% or more as compared to the corresponding activity in the same subject prior to treatment or compared to the corresponding activity in other subjects not receiving the combination therapy. An exemplary compound includes PRX-03140 (Predix), which is a highly selective, small-molecule agonist.

As used herein, by "anti-inflammatory agent' is meant a compound that reduces or eliminates inflammation. In some embodiments, the compound reduces inflammation by at least or about 10%, 20%, 30%, 40%, 50%, 60%, 70%, 80%, 90%, 95% or 100%. An exemplary compound includes VP-025 (Vasogen), which is a bilayered phospholipid microparticle that interacts with macrophages and other cells of the immune system, eliciting an anti-inflammatory response.

As used herein, by "combination therapy" is meant a first therapy that includes one or more compound which is 2,8-dimethyl-5-(2-(6-methyl-3-pyridyl)ethyl)-2,3,4,5-tetrahydro-1H-pyrido[4,3-b]indole or pharmaceutically acceptable salts thereof in conjunction with a second therapy that includes donepezil (or pharmaceutically acceptable salts thereof). Administration in "conjunction with" another compound includes administration in the same or different composition, either sequentially, simultaneously, or continuously. In some embodiments, the combination therapy includes one or more compound which is 2,8-dimethyl-5-(2-(6-methyl-3-pyridyl)ethyl)-2,3,4,5-tetrahydro-1H-pyrido[4,3-b]indole or pharmaceutically acceptable salts thereof and one or more compounds that is donepezil or pharmaceutically acceptable salts thereof. In some variations, the combination therapy optionally includes one or more pharmaceutically acceptable carriers or excipients, non-pharmaceutically active compounds, and/or inert substances.

As used herein, by "pharmaceutically active compound," "pharmacologically active compound" or "active ingredient" is meant a chemical compound that induces a desired effect, *e.g*., treating and/or preventing and/or delaying the onset and/or the development of Alzheimer's disease.

The term "effective amount" intends such amount of a compound (*e.g*., a component of a combination therapy of the invention) or a combination therapy, which in combination with its parameters of efficacy and toxicity, as well as based on the knowledge of the practicing specialist should be effective in a given therapeutic form. As is understood in the art, an effective amount may be in one or more doses, *i.e.,* a single dose or multiple doses may be required to achieve the desired treatment endpoint. In some embodiments, the amount of the first therapy, the second therapy, or the combined therapy is an amount sufficient to increase the amount or activity of acetylcholine, reduce an activity of an acetylcholinesterase or reduce an activity of an NMDA receptor.
In some embodiments, one or more of these activities changes by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. Standard methods can be used to measure the magnitude of this effect, such as *in vitro* assays with purified enzyme, cell-based assays, animal models, or human testing.

As is understood in the clinical context, an effective dosage of a drug, compound or pharmaceutical composition that contains a compound which is 2,8-dimethyl-5-(2-(6-methyl-3-pyridyl)ethyl)-2,3,4,5-tetrahydro-1H-pyrido[4,3-b]indole may be achieved in conjunction with donepezil or pharmaceutical composition that contains donepezil or pharmaceutically acceptable salts. Thus, an effective amount may be considered in the context of administering one or more therapeutic agents, and a single agent may be considered to be given in an effective amount if, in conjunction with one or more other agents, a desirable or beneficial result may be or is achieved. The compounds in a combination therapy of the invention may be administered sequentially, simultaneously, or continuously using the same or different routes of administration for each compound. Thus, an effective amount of a combination therapy includes an amount of the first therapy and an amount of the second therapy that when administered sequentially, simultaneously, or continuously produces a desired outcome. Suitable doses of any of the coadministered compounds may optionally be lowered due to the combined action (*e.g*., additive or synergistic effects) of the compounds.

In various embodiments, treatment with the combination of the first and second therapies may result in an additive or even synergistic (*e.g*., greater than additive) result compared to administration of either therapy alone. In some embodiments, a lower amount of each pharmaceutically active compound is used as part of a combination therapy compared to the amount generally used for individual therapy. Preferably, the same or greater therapeutic benefit is achieved using a combination therapy than by using any of the individual compounds alone. In some embodiments, the same or greater therapeutic benefit is achieved using a smaller amount (*e.g*., a lower dose or a less frequent dosing schedule) of a pharmaceutically active compound in a combination therapy than the amount generally used for individual therapy. Preferably, the use of a small amount of pharmaceutically active compound results in a reduction in the number, severity, frequency, or duration of one or more side-effects associated with the compound.

A "therapeutically effective amount" refers to an amount of a compound or a combination therapy sufficient to produce a desired therapeutic outcome (*e.g*., reducing the severity or duration of, stabilizing the severity of, or eliminating one or more symptoms of Alzheimer's disease). For therapeutic use, beneficial or desired results include, *e.g*., clinical results such as inhibiting or suppressing the formation of amyloid plaques, reducing, removing, or clearing amyloid plaques, improving cognition or reversing cognitive decline, sequestering soluble Aβ peptide circulating in biological fluids, decreasing one or more symptoms resulting from the disease (biochemical, histologic and/or behavioral), including its complications and intermediate pathological phenotypes presenting during development of the disease, increasing the quality of life of those suffering from the disease, decreasing the dose of other medications required to treat the disease, enhancing effect of another medication, delaying the progression of the disease and/or prolonging survival of patients.

A "prophylactically effective amount" refers to an amount of a compound or a combination therapy sufficient to prevent or reduce the severity of one or more future symptoms of Alzheimer's disease when administered to an individual who is susceptible and/or who may develop Alzheimer's disease. For prophylactic use, beneficial or desired results include, e.g., results such as eliminating or reducing the risk, lessening the severity, or delaying the onset of the disease, including biochemical, histologic and/or behavioral symptoms of the disease, its complications and intermediate pathological phenotypes presenting during development of the disease.

The term ``simultaneous administration," as used herein, means that a first therapy and second therapy in a combination therapy are administered with a time separation of no more than about 15 minutes, such as no more than about any of 10, 5, or 1 minutes. When the compounds are administered simultaneously, the first and second therapies may be contained in the same composition (*e.g*., a composition comprising both a compound which is a 2,8-dimethyl-5-(2-(6-methyl-3-pyridyl)ethyl)-2,3,4,5-tetrahydro-1H-pyrido[4,3-b]indole and donepezil (or a pharmaceutically acceptable salt thereof) or in separate compositions (*e.g*., a compound which is 2,8-dimethyl-5-(2-(6-methyl-3-pyridyl)ethyl)-2,3,4,5-tetrahydro-1H-pyrido[4,3-b]indole is contained in one composition and donepezil or a pharmaceutically acceptable salt thereof is contained in another composition).

As used herein, the term "sequential administration" means that the first therapy and second therapy in a combination therapy are administered with a time separation of more than about 15 minutes, such as more than about any of 20, 30, 40, 50, 60 or more minutes. Either the first therapy or the second therapy may be administered first. The first and second therapies are contained in separate compositions, which may be contained in the same or different packages or kits.

The term "controlled release" refers to a drug-containing formulation or fraction thereof in which release of the drug is not immediate, *i.e*., with a "controlled release" formulation, administration does not result in immediate release of the drug into an absorption pool.

As used herein, by "pharmaceutically acceptable" or "pharmacologically acceptable" is meant a material that is not biologically or otherwise undesirable, *e.g*.., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

### Methods for Treating Alzheimer's disease

The first therapy described herein is a hydrogenated pyrido [4,3-b] indole which may be used to treat, prevent and/or delay the onset and/or the development of Alzheimer's disease in mammals, such as humans. As illustrated in Example 1, dimebon is a well-tolerated drug that improved cognition, function and behavior in patients with mild-moderate Alzheimer's disease.

Combination therapies that include a hydrogenated pyrido [4,3-b] indole and another compound for Alzheimer's disease may have enhanced activity for treating, preventing and/or delaying the onset and/or development of Alzheimer's disease. In particular, these combination therapies include one or more compound which is 2,8-dimethyl-5-(2-(6-methyl-3-pyridyl)ethyl)-2,3,4,5-tetrahydro-1H-pyrido[4,3-b]indole or a pharmaceutically acceptable salts thereof in conjunction with one or more other compounds which is donepezil or pharmaceutically acceptable salts thereof. Methods that use such combination therapies may result in an additive or even synergistic (*e.g*., greater than additive) result compared to administration of either therapy alone. Improved results are expected using combination therapies because hydrogenated pyrido [4,3-b] indoles seem to act at least in part through a mechanism that differs from that of other compounds useful for Alzheimer's disease. For example, dimebon has a neuroprotective effect that inhibits neuronal cell death. This disease-modifying effect is different from the improvement in symptoms without a decrease in cell death that results from some acetylcholinesterase inhibitors or NMDA receptor antagonists. The neuroprotective effect of dimebon is also likely to involve a different mechanism from that of VEGF. In particular, the activation of Flk-1 by VEGF for its neuroprotective effect requires much of the VEGF protein. Thus, the small molecule dimebon is unlikely to directly activate Flk-1 as part of its ability to inhibit neuronal cell death. Additionally, the magnitude of the effect of dimebon in treating Alzheimer's disease as reported in Example 1 is greater than that of other Alzheimer's disease compounds. In summary, the neuroprotective actions of dimebon indicate a mechanism of action distinct from other classes of Alzheimer's disease drugs (*e.g*., acetylcholinesterase inhibitors and NDMA receptor antagonists).
Thus, administering a hydrogenated pyrido [4,3-b] indole such as dimebon in conjunction with another Alzheimer's disease therapy is expected to result in enhanced results due to the combined effect of the therapies through multiple mechanisms that are useful for treating, preventing and/or delaying the onset and/or development of Alzheimer's disease. For example, the combination therapy may result in an inhibition of neuronal cell death that is not achieved without the use of a hydrogenated pyrido [4,3-b] indole. Also, a combination therapy may result in a longer duration of improvement in symptoms since the effectiveness of some acetylcholinesterase inhibitors or NMDA receptor antagonists when administered alone decreases over time. Exemplary methods for determining the ability of combination therapies to treat, stabilize, delay and/or prevent Alzheimer's disease are described in Examples 1-3.

Combination therapies are desirable because of the improved clinical results that can be obtained. Also, combination therapies may require lower doses of the individual therapies than would be necessary if the individual therapies were given alone. This decreased dosage may reduce side-effects associated with the therapies. Thus, in some embodiments, a lower amount of each pharmaceutically active compound is used as part of a combination therapy compared to the amount generally used for individual therapy. In some embodiments, the same or greater therapeutic benefit is achieved using a smaller amount (*e.g*., a lower dose or a less frequent dosing schedule) of a pharmaceutically active compound in a combination therapy than the amount generally used for individual therapy. Preferably, the use of a small amount of pharmaceutically active compound results in a reduction in the number, severity, frequency or duration of one or more side-effects associated with the compound.

Thus, the present invention provides a compound for use in a variety of methods using combination therapies, such as those described in the "Brief Summary of the Invention" and elsewhere in this disclosure. The methods employ the compounds described herein. For example, in one embodiment, the present invention provides a compound such as dimebon for use in a method as defined in claim 1. The method may be a method for treating Alzheimer's disease in a patient in need thereof; a method of decreasing the intensity or severity of the symptoms of Alzheimer's disease in an individual who is diagnosed with Alzheimer's disease; a method of increasing the survival time of an individual diagnosed with Alzheimer's disease; or a method of enhancing the quality of life of an individual diagnosed with Alzheimer's disease.

In one embodiment, the method is a method of delaying the onset and/or development of Alzheimer's disease in an individual who is considered at risk for developing Alzheimer's disease (*e.g*., an individual whose one or more family members have had Alzheimer's disease or an individual who has been diagnosed as having a genetic mutation associated with Alzheimer's disease). In one embodiment, the method is a method of delaying the onset and/or development of Alzheimer's disease in an individual who is genetically predisposed to developing Alzheimer's disease. In one embodiment, the method is a method of delaying the onset and/or development of Alzheimer's disease in an individual having a mutated or abnormal gene associated with Alzheimer's disease (*e.g*., an APP mutation, a presenilin mutation and/or an ApoE4 allele) but who has not been diagnosed with Alzheimer's disease.
In various embodiments of methods of delaying the onset of Alzheimer's disease, the method delays or prevents one or more biochemical, histologic and/or behavioral symptoms of the disease, one or more complications of the disease, and/or one or more intermediate pathological phenotypes presenting during development of the disease.

In one embodiment, the method is a method of preventing Alzheimer's disease in an individual who is genetically predisposed to developing Alzheimer's disease or who has a mutated or abnormal gene associated with Alzheimer's disease but who has not been diagnosed with Alzheimer's disease. In one embodiment, the method is a method of preventing the onset and/or development of Alzheimer's disease in an individual who is not identified as genetically predisposed to developing Alzheimer's disease. In one variation, the present invention provides use of a compound 2,8-dimethyl-5-(2-(6-methyl-3-pyridyl)ethyl)-2,3,4,5-tetrahydro-1H-pyrido[4,3-b]indole or pharmaceutically acceptable salt thereof in the manufacture of a medicament for use in any of the above methods, *e.g*., treating and/or preventing and/or delaying the onset or development of Alzheimer's disease in a human.

In various embodiments of any of the above methods, the second therapy includes a compound that increases the amount or activity of acetylcholine (*e.g*., an acetylcholinesterase inhibitor). In various embodiments, the first therapy is dimebon; the acetylcholinesterase inhibitor is Aricept.

### Compounds for Use in the Methods, Combination Therapies, Formulations, Kits and Inventions Discloses Herein

The combination therapies described herein employ more than one therapeutic agent. The combination therapies described intend and include all available combinations described herein, *e.g*., by selecting one or more compound described under the heading "hydrogenated pyrido[4,3-b]indoles for use in a first therapy" and one or more compound described under the heading "compounds for use in a second or additional therapy." For instance, the compound dimebon may be selected from under the heading "hydrogenated pyrido[4,3-b]indoles for use in a first therapy" and combined with any one or more compound described under the heading "compounds for use in a second or additional therapy." It is recognized that a combination therapy may include two or more therapeutic agents. A combination therapy can include one compound described under the heading "hydrogenated pyrido[4,3-b]indoles for use in a first therapy" (e.g., dimebon) and one or more compounds as described under the heading "compounds for use in a second or additional therapy." Each such combination is described hereby to the same extent as if each and every combination were specifically and individually listed. For example, a composition may consist of dimebon and Namenda and Arecept.

For each compound listed herein by tradename, e.g., Namenda, such listing intends and includes the chemical entity of the active ingredient and pharmaceutically acceptable salts thereof and is not limited to compounds marketed only under the stated tradename; compounds that are identical to or bioequivalent to the listed tradename are included and intended.

When reference to organic residues or moieties having a specific number of carbons is made, unless clearly stated otherwise, it intends all geometric isomers thereof. For example, "butyl" includes n-butyl, sec-butyl, isobutyl and t-butyl; "propyl" includes n-propyl and isopropyl.

The term "alkyl" intends and includes linear, branched or cyclic hydrocarbon structures and combinations thereof. Preferred alkyl groups are those having 20 carbon atoms (C20) or fewer. More preferred alkyl groups are those having fewer than 15 or fewer than 10 or fewer than 8 carbon atoms.

The term "lower alkyl" refers to alkyl groups of from 1 to 5 carbon atoms. Examples of lower alkyl groups include methyl, ethyl, propyl, isopropyl, butyl, s- and t-butyl and the like. Lower alkyl is a subset of alkyl.

The term "aryl" refers to an unsaturated aromatic carbocyclic group of from 6 to 14 carbon atoms having a single ring (*e.g*., phenyl) or multiple condensed rings (*e.g.,* naphthyl or anthryl) which condensed rings may or may not be aromatic (*e.g*., 2-benzoxazolinone, 2H-1,4-benzoxain-3(4H)-one-7-yl), and the like. Preferred aryls includes phenyl and naphthyl.

The term "heteroaryl" refers to an aromatic carbocyclic group of from 2 to 10 carbon atoms and 1 to 4 heteroatoms selected from oxygen, nitrogen and sulfur within the ring. Such heteroaryl groups can have a single ring (*e.g*., pyridyl or furyl) or multiple condensed rings (*e.g*., indolizinyl or benzothienyl). Examples of heteroaryl residues include, *e.g*., imidazolyl, pyridinyl, indolyl, thiopheneyl, thiazolyl, furanyl, benzimidazolyl, quinolinyl, isoquinolinyl, pyrimidinyl, pyrazinyl, tetrazolyl and pyrazolyl.

The term "aralkyl" refers to a residue in which an aryl moiety is attached to the parent structure via an alkyl residue. Examples are benzyl, phenethyl and the like.

The term "heteroaralkyl" refers to a residue in which a heteroaryl moiety is attached to the parent structure via an alkyl residue. Examples include furanylmethyl, pyridinylmethyl, pyrimidinylethyl and the like.

The term "substituted heteroaralkyl" refers to heteroaryl groups which are substituted with from 1 to 3 substituents, such as residues selected from the group consisting of hydroxy, alkyl, alkoxy, alkenyl, alkynyl, amino, aryl, carboxyl, halo, nitro and amino.

The term "substituted aralkyl" refers to aralkyl groups which are substituted with from 1 to 3 substituents, such as residues selected from the group consisting of hydroxy, alkyl, alkoxy, alkenyl, alkynyl, amino, aryl, carboxyl, halo, nitro and amino.

The term "halo" or "halogen" refers to fluoro, chloro, bromo and iodo.

### Hydrogenated pyrido [4,3-b] indoles

According to the claimed invention the first therapy comprises a hydrogenated pyrido [4,3-b] indole which is 2,8-dimethyl-5-(2-(6-methyl-3-pyridyl)ethyl)-2,3,4,5-tetrahydro-1H-pyrido[4,3-b]indole or a pharmaceutically acceptable salt thereof.

The hydrogenated pyrido [4,3-b] indole can be in the form of pharmaceutically acceptable salts thereof, which are readily known to those of skill in the art. The pharmaceutically acceptable salts include pharmaceutically acceptable acid salts. Examples of particular pharmaceutically acceptable salts include hydrochloride salts or dihydrochloride salts. In a particular variation, the hydrogenated pyrido [4,3-b] indole is a pharmaceutically acceptable salt of 2,8-dimethyl-5-(2-(6-methyl-3-pyridyl)ethyl)-2,3,4,5-tetrahydro-1H-pyrido[4,3-b]indole, such as 2,8-dimethyl-5-(2-(6-methyl-3-pyridyl)ethyl)-2,3,4,5-tetrahydro-1H-pyrido[4,3-b]indole dihydrochloride (dimebon).

The compound for use in the systems and methods may be 2,8-dimethyl-5-(2-(6-methyl-3-pyridyl)ethyl-2,3,4,5-tetrahydro-1H-pyrido[4,3-b]indole or any pharmaceutically acceptable salt thereof, such as an acid salt, a hydrochloride salt or a dihydrochloride salt thereof.
Synthesis of these compounds is described in the article by A.N. Kost, M.A. Yurovskaya, T.V. Mel'nikova, in Chemistry of heterocyclic compounds, 1973, N 2, p. 207-212.

### Compounds for use in a second or additional therapy

The compound for use in the second therapy is donepezil or pharmaceutically acceptable salt thereof.

### Acetylcholinesterase inhibitors

The second therapy herein may include use of further acetylcholinesterase inhibitors. Pathological changes in dementia of the Alzheimer's type involve cholinergic neuronal pathways that project from the basal forebrain to the cerebral cortex and hippocampus. These pathways are thought to be intricately involved in memory, attention, learning and other cognitive processes. It is generally believed that cholinesterase inhibitors exert their therapeutic effect by enhancing cholinergic function. This result is accomplished by increasing the concentration of acetylcholine through reversible inhibition of its hydrolysis by acetylcholinesterase. Thus, since the cholinergic system is impaired in Alzheimer's disease patients, acetylcholinesterase inhibitors are useful for improving symptoms of Alzheimer's disease by inhibiting the ability of acetylcholinesterase to hydrolyze acetylcholine (Volger, "Alternatives in the treatment of memory loss in patient with Alzheimer's disease," Clinical Pharmacy, (6): 447-56, June 10, 1991). An exemplary and non-limiting list of acetylcholinesterase inhibitors includes Aricept (donepezil), Exelon (rivastigmine tartrate), Razadyne (Reminyl, galantamine) and Tacrine (Cognex, 9-amino-1,2,3,4-tetrahydroacridine hydrochloride). Ladostigil (TEVA, TV3326) has acetylcholinesterase inhibitory activity as well as brain-selective MAO-inhibitory activity and neuroprotective activity. Ladostigil reduces apoptotic cell death (human neuroblastoma SK-N-SH) in response to long term culture, reduces apoptosis-induced levels of holo-APP protein and increases phospho PKC and the release of the nonamyloidogenic amyloid protein (Yogev-Falach et al., 2006 FASEB J Bar*;* Am et al., 2004 J Neurochem, 89:1119-25).

In one variation, the acetylcholine esterase inhibitor is galanthamine shown below as formula (Z): 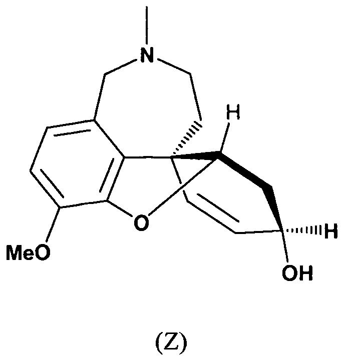 which may be administered in any available chemical or physical form. Galanthamine can be administered as a pharmaceutically acceptable salt, such as a hydrobromide, hydrochloride, methylsulfate or methiodide salt thereof. In one variation galanthamin is administered with a pharmaceutically-acceptable acid addition salt. In one variation, galanthamine is administered in a pharmaceutically acceptable carrier, characterized in that said carrier comprises a spray-dried mixture of lactose monohydrate and microcrystalline cellulose (75:25) as a diluent, and a disintegrant. In one variation galanthamine is administered using either lactose anhydrous or lactose monohydrate as diluent, and either powdered cellulose or microcrystalline cellulose as disintegrant. Galanthamine may be administered in a tablet comprising as an active ingredient a therapeutically effective amount of galanthamine hydrobromide (1:1) and a pharmaceutically acceptable carrier, characterized in that said carrier comprises a spray-dried mixture of lactose monohydrate and microcrystalline cellulose (75:25) as a diluent, and a disintegrant, which may be an insoluble or poorly soluble cross-linked polymer disintegrant, such as, for example, crospolyvidone or croscarmellose. Said tablets have a dissolution of at least 80% after 30 minutes. In one variation the tablet further comprises a glidant and a lubricant. The glidant may be colloidal anhydrous silica and the lubricant may be magnesium stearate. In one variation, galanthamine may be formulated in a fast-dissolving galanthamine hydrobromide (1:1) tablet made by (i) dry blending the active ingredient, an insoluble or poorly soluble cross-linked polymer disintegrant and an optional glidant with a diluent comprising a spray-dried mixture of lactose monohydrate and microcrystalline cellulose (75:25); (ii) optionally mixing a lubricant with the mixture obtained in step (i); (iii) compressing the mixture obtained in step (i) or in step (ii) in the dry state into a tablet; and (iv) optionally film-coating the tablet obtained in step (iii). Methods of making and using galanthamine and formulations comprising galanthamine are described, *e.g*., in U.S. Patent Nos. 4,663,318, 6,099,863 and 6,358,527.

In one variation, the acetylcholine esterase inhibitor is a compound of the formula (Y): 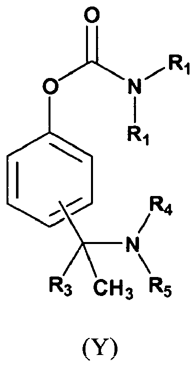 wherein R₁ is hydrogen, lower alkyl, cyclohexyl, allyl or benzyl; R₂ is hydrogen, methyl, ethyl or propyl, or R₁ and R₂ together with the nitrogen to which they are attached form a morpholino or piperidino radical; R₃ is hydrogen or lower alkyl; R₄ and R₅ are the same or different and each is a lower alkyl, and the dialkylaminoalkyl group is in the meta, ortho or para position, in free base or a pharmacologically acceptable salt thereof, such as a pharmaceutically acceptable acid addition salt thereof. In one variation the dialkylaminoalkyl group is in the meta position, and R₄ and R₅ are both methyl. In one variation R₁ and R₃ are each H and R₂, R₄ and R₅ are each methyl, which compound is Miotine®. In one variation R₁ and R₂ are methyl, R₃ is H and R₄ and R₅ are methyl. In one variation, R₁ is H and R₂, R₃, R₄ and R₅ are -CH₃. In one variation, the acetylcholine esterase inhibitor is selected from the group consisting ofN-ethyl-3-[1-(dimethylamino)ethyl]phenyl carbamate, N-propyl-3[1-(dimethylamino)ethyl]phenyl carbamate, N-allyl-3-[1-(dimethylamino)ethyl]phenyl carbamate, N-ethyl, N-methyl-3[1-(dimethylamino)ethyl]phenyl carbamate, N,N-diethyl-3[1-(dimethylamino)ethyl]phenyl carbamate, N-butyl-3-[1-(dimethylamino)ethyl]phenyl carbamate, N-methyl, N-propyl-3[1-(dimethylamino)ethyl]phenyl carbamate and N-ethyl, N-methyl-3[1-(dimethylamino)isopropyl]phenyl carbamate or pharmacologically acceptable salts thereof, such as the acetate, salicylate, fumarate, phosphate, sulphate, maleate, succinate, citrate, tartrate, propionate and butyrate salts thereof. In one variation the acetylcholine esterase inhibitor is N-cyclohexyl-3-[1-(dimethylamino)ethyl]phenyl carbamate or pharmacologically acceptable salts thereof. In one variation the acetylcholine esterase inhibitor is N-allyl-3-[1-(dimethylamino)ethyl]phenyl carbamate and pharmacologically acceptable salts thereof. In one variation the acetylcholine esterase inhibitor is N-ethyl, N-methyl-3-[1-(dimethylamino)ethyl]phenyl carbamate and pharmacologically acceptable salts thereof. In one variation the compound is (S)-N-ethyl-3-[(1-dimethylamino)ethyl]-N-methyl-phenyl-carbamate of formula (X): 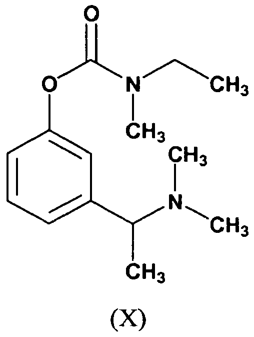 in free base or acid addition salt form. In one variation the compound is the hydrogen tartrate salt of (S)-N-ethyl-3-[(1-dimethylamino)ethyl]-N-methyl-phenyl-carbamate. In one variation the compound is (S)-[N-ethyl-3-[(1-dimethylamino)ethyl]-N-methyl-phenyl-carbamate] enantiomer of formula (X) substantially free of its (R) isomer. A composition comprising the (S) isomer may be 99% free of the (R) isomer or about 95 % free of the (R) isomer or about 90% free of the (R) isomer or about 80% free of the (R) isomer. In one variation the compound is a racemic mixture (±)-N-ethyl-3-[(1-dimethylamino)ethyl]-N-methyl-phenyl-carbamate in form of its hydrochloride. Methods of making and using compounds of this paragraph and formulations thereof are described in U.S. Patent Nos. 4,948,807, 5,602,176 and 6,372,760.

In one variation, the acetylcholine esterase inhibitor is a compound of the formula (W) 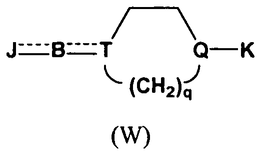 wherein J is: (a) a group, substituted or unsubstituted, selected from the group consisting of (1) phenyl, (2) pyridyl, (3) pyrazyl, (4) quinolyl, (5) cyclohexyl, (6) quinoxalyl and (7) furyl; (b) a monovalent or divalent group, in which the phenyl may have a substituent(s), selected from the group consisting of (1) indanyl, (2) indanonyl, (3) indenyl, (4) indenonyl, (5) indanedionyl, (6) tetralonyl, (7) benzosuberonyl, (8) indanolyl and (9) C₆ H₅ --CO--CH(CH₃)--;(c) a monovalent group derived from a cyclic amide compound; (d) a lower alkyl or (e) a group of R²¹ --CH=CH-- in which R²¹ is hydrogen or a lower alkoxycarbonyl; and wherein B is -(CHR²²)ᵣ-, -CO-(CHR²²)ᵣ-, -NR⁴-(CHR²²)ᵣ-, R⁴ being hydrogen, a lower alkyl, an acyl, a lower alkylsulfonyl, phenyl, a substituted phenyl, benzyl or a substituted benzyl, -CO-NR⁵-(CHR²²)ᵣ-, R⁵ being hydrogen, a lower alkyl or phenyl,-CH=CH-(CHR²²)ᵣ-, -OCOO-(CHR²²)ᵣ-,-OOC-NH-(CHR²²)ᵣ-,-NH-CO-(CHR²²)ᵣ-,-CH₂-CO-NH-(CHR²²)ᵣ-, -(CH₂)₂-NH-(CHR²²)ᵣ-, -CH(OH)-(CHR²²)ᵣ-, r being zero or an integer of 1 to 10, R²² being hydrogen or methyl so that one alkylene group may have no methyl branch or one or more methyl branch, =(CH--CH=CH)b-, b being an integer of 1 to 3, =CH-(CH₂)_{c}-, c being zero or an integer of 1 to 9, =(CH-CH)_{d}=, d being zero or an integer of 1 to 5; -CO-CH=CH-CH₂-, -CO-CH₂-CH(OH)-CH₂-, -CH(CH₃)-CO-NH-CH₂-, -CH=CH-CO-NH-(CH₂)₂-, -NH-, -O-, -S-, a dialkylaminoalkylcarbonyl or a lower alkoxycarbonyl; T is a nitrogen or carbon; Q is nitrogen, carbon or N→O; q is an integer of 1 to 3; K is hydrogen, phenyl, a substituted phenyl, an arylalkyl in which the phenyl may have a substituent, cinnamyl, a lower alkyl, pyridylmethyl, a cycloalkylalkyl, adamantanemethyl, furylmenthyl, a cycloalkyl, a lower alkoxycarbonyl or an acyl; and ----- shows a single bond or a double bond. In one variation J is (a) or (b). In one variation monovalent groups of (b) are (2), (3) or (5). In one variation divalent groups of (b) are (2). In one variation B is -(CHR²²)ᵣ-, =(CH-CH=CH)b-, =CH-(CH₂)c- or =(CH-CH)d=. In one variation groups of (B) may be connected with (b) of J, in particular (2) of (b). In one variation Q is nitrogen, T is carbon and q is 1 or 3; and Q is carbon, T is nitrogen and q is 2. In one variation Q is nitrogen, T is carbon and q is 2. In one variation K is a phenylalkyl or a phenylalkyl having a substituent(s) on the phenyl. In one variation a compound for use in the second therapy is selected from the group consisting of: 1-benzyl-4-((5,6-dimethoxy-1-indanon)-2-yl)methylpiperidine, 1-benzyl-4-((5,6-dimethoxy-1-indanon)-2-ylidenyl)methylpiperidine, 1-benzyl-4-((5-methoxy-1-indanon)-2-yl)methylpiperidine, 1-benzyl-4-((5,6-diethoxy-1-indanon)-2-yl)methylpiperidine, 1-benzyl-4-((5,6-methnylenedioxy-1-indanon)-2-yl)methylpiperidine, 1-(m-nitrobenzyl)-4-((5,6-dimethoxy-1-indanon)-2-yl)methylpiperidine, 1-cyclohexymethyl-4-((5,6-dimethoxy-1-indanon)-2-yl)methylpiperidine, 1-(m-florobenzyl)-4-((5,6-dimethoxy-1-indanon)-2-yl)methylpiperidine, 1-benzyl-4-((5,6-dimethoxy-1-indanon)-2-yl)propylpiperidine, 1-benzyl-4-((5-isopropoxy-6-methoxy-1-indanon)-2-yl)methylpiperidine and 1-benzyl-4-((5,6-dimethoxy-1-oxoindanon)-2-yl)propenylpiperidine. Additional compounds for use in the second therapy include compounds in U.S. Patent No. 4,895,841 such as the compounds disclosed in columns 3-12 and Tables 4-9 thereof. A compound for use in the second therapy may be a compound of the general formula (U): 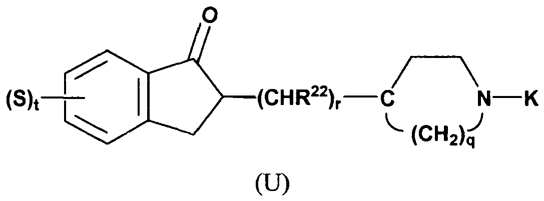 wherein r is an integer of 1 to 10 , R²² is hydrogen or methyl, and the R²² radicals can be the same or different when r is from 2 to 10; K is phenylalkyl or phenylalkyl having a substituent on the phenyl ring; S is hydrogen or a substituent on the phenyl ring, and t is an integer of 1 to 4, with the proviso that (S)ₜ can be a methylenedioxy group or an ethylenedioxy group joined to two adjacent carbon atoms of the phenyl ring; and q is an integer of 1 to 3, or a pharmacologically acceptable salt thereof. In one variation q is 2. In one variation K is benzyl, m-nitrobenzyl or m-fluorobenzyl. In one variation K is benzyl. In one variation S is lower alkyl having 1 to 6 carbon atoms or lower alkoxy having 1 to 6 carbon atoms. In one variation S is methoxy and t is an integer of from 1 to 3. In one variation r is an integer of 1 to 3. In one variation the compound is 1-benzyl-4-((5,6-dimethoxy-1-indanon)-2-yl)methylpiperidine or a pharmacologically acceptable salt thereof. In one variation the compound is selected from the group consisting of: 1-benzyl-4-((5-methoxy-1-indanon)-2-yl)methylpiperidine, 1-benzyl-4-((5,6-diethoxy-1-indanon)-2-yl)methylpiperidine, 1-benzyl-4-((5,6-methylenedioxy-1-indanon)-2-yl)methylpiperidine, 1-(m-nitrobenzyl)-4-((5,6-dimethoxy-1-indanon)-2-yl)methylpiperidine, 1-(m-fluorobenzyl)-4-((5,6-dimethoxy-1-indanon)-2-yl)methylpiperidine, 1-benzyl-4-((5,6-dimethoxy-1-indanon)-2-yl)propylpiperidine and 1-benzyl-4-((5-isopropoxy-6-methoxy-1-indanon)-2-yl)methylpiperidine or a pharmacologically acceptable salt thereof. Methods of making and using compounds such as compounds of the formula (W) and (U) are described in U.S. Patent No. 4,895,841, which is incorporated herein by reference in its entirety.
The second therapy comprises donepezil. In one variation, the second therapy comprises donepezil and an organic acid selected from the group consisting of tosyllic acid, mesyllic acid, benzoic acid, salicylic acid, tartaric acid, citric acid and combinations thereof, wherein the organic acid is not added to form a salt. In one variation the amount of organic acid is 0.2 to 5 parts by weight to 1 part by weight of donepezil. In one variation, the amount of organic acid is 0.2 to 2 parts by weight to 1 part by weight of donepezil.

In one variation, a compound of the second therapy is the compound donepezil hydrochloride, 1-benzyl-4-[(5,6-dimethoxy-1-indanon)-2-yl]methylpiperdine hydrochloride, in any form, including a form of polymorphs selected from the group consisting of (II), (IV) and (V), each polymorph being specified by peaks at below shown diffraction degrees with the below shown intensity in terms of I/Iₒ in x-ray powder diffraction pattern and the below shown absorption peaks in infrared absorption spectra in potassium bromide in terms of reciprocal centimeters:

| Polymorph (II) | |
|---|---|
| Peaks in the powder X-ray diffraction pattern are: Diffraction angles | |
| Intensity | |
| (20, °) | (I/I °) |
| 10.10 | 76 |
| 12.64 | 14 |
| 15.74 | 85 |
| 15.82 | 86 |
| 16.20 | 100 |
| 16.46 | 87 |
| 17.40 | 50 |
| 17.50 | 48 |
| 17.88 | 31 |
| 18.36 | 28 |
| 18.58 | 51 |
| 18.66 | 46 |
| 19.48 | 42 |
| 20.18 | 81 |
| 20.80 | 36 |
| 22.26 | 45 |
| 23.38 | 86 |
| 23.52 | 59 |
| 24.06 | 34 |
| 24.32 | 55 |
| 25.14 | 44 |
| 25.44 | 50 |
| 25.72 | 39 |
| 25.96 | 35 |
| 26.14 | 25 |
| 28.06 | 25 |
| 28.20 | 34 |
| 28.38 | 34 |

Wave numbers (cm⁻¹⁾ if infrared absorption spectra in potassium bromide are:
- 560.1, 698.9, 749.1, 846.2, 947.6, 1036.1, 1119.3, 1222.7,
- 1266.4, 1318.7, 1364.1, 1458.3, 1500.9, 1522.3, 1534.0, 1542.6,
- 1560.2, 1570.3, 1592.0, 1637.0, 1647.9, 1654.4, 1689.5, 1718.3,
- 1734.7, 1751.7, 1773.9, 1793.8, 1830.7, 1846.0, 1870.1, 2354.1,
- 2489.9, 2927.9, 3448.1 cm⁻¹

| Polymorph (IV) | |
|---|---|
| Peaks in the powder X-ray diffraction pattern are: Diffraction angles | |
| Intensity | |
| (20, °) | (I/I°) |
| 9.64 | 11 |
| 10.92 | 11 |
| 12.46 | 63 |
| 12.72 | 17 |
| 13.86 | 27 |
| 14.42 | 12 |
| 17.36 | 100 |
| 18.54 | 39 |
| 19.90 | 37 |
| 21.18 | 35 |
| 21.74 | 39 |
| 22.48 | 60 |
| 22.96 | 36 |
| 24.10 | 17 |
| 25.28 | 70 |
| 28.00 | 27 |
| 28.50 | 27 |

Wave numbers (cm⁻¹⁾ of infrared absorption spectra in potassium bromide are:
- 561.5, 709.0, 766.2, 786.3, 804.9, 857.0, 944.3, 979.3, 1041.5,
- 1118.7, 1264.6, 1318.7, 1364.1, 1458.1, 1499.2, 1542.5, 1560.1,
- 1588.1, 1636.6, 1647.8, 1654.3, 1684.3, 1718.2, 1734.4, 1751.4,
- 1773.7, 1793.5, 1830.5, 1845.8, 1870.1, 2344.8, 2369.3, 2719.2,
- 2922.9, 3324.0 cm⁻¹

| Polymorph (V) | |
|---|---|
| Diffraction angles | |
| Intensity | |
| (20, °) | (I/I°) |
| 6.58 | 7 |
| 6.86 | 27 |
| 10.12 | 32 |
| 12.54 | 33 |
| 12.90 | 43 |
| 13.64 | 64 |
| 15.58 | 27 |
| 17.22 | 69 |
| 18.44 | 72 |
| 18.96 | 19 |
| 19.30 | 25 |
| 19.64 | 19 |
| 19.74 | 25 |
| 20.30 | 19 |
| 20.46 | 17 |
| 21.10 | 15 |
| 21.96 | 100 |
| 22.24 | 32 |
| 24.22 | 63 |
| 24.66 | 96 |
| 25.36 | 60 |
| 26.14 | 15 |
| 26.82 | 44 |
| 27.52 | 24 |
| 27.96 | 15 |
| 28.20 | 49 |
| 29.58 | 13 |
| 29.66 | 17 |
| 29.76 | 17 |

Wave numbers (cm⁻¹⁾ of infrared absorption spectra in potassium bromide are:
- 506.5, 559.7, 594.4, 698.0, 740.8, 805.1, 861.9, 948.5, 972.1,
- 1039.9, 1120.8, 1220.7, 1264.8, 1314.6, 1364.1, 1458.0, 1499.5,
- 1542.5, 1560.2, 1592.1, 1692.9, 2500.1, 2924.2, 2998.9, 3422.1, cm⁻¹.
In one variation the Donepezil hydrochloride is in the form of polymorph (II). In one variation the Donepezil hydrochloride is in the form of polymorph (IV). In one variation the Donepezil hydrochloride is in the form of polymorph (V). Methods of making and using donepezil and polymorphs thereof are described in U.S. Patent No. 5,985,864.

In one variation, the compound of a second therapy is the compound donepezil hydrochloride, 1-benzyl-4-[(5,6-dimethoxy-1-indanon)-2-yl]methylpiperidine hydrochloride, in the form of a polymorph being specified by peaks at below shown diffraction degrees with the below shown intensity in terms of I/Iₒ in X-ray powder diffraction pattern and the below shown absorption peaks in infrared absorption spectra in potassium bromide in terms of reciprocal centimeters: Polymorph (III) Exhibiting peaks in the powder X-ray diffraction pattern as follows:

| Diffraction angles | |
|---|---|
| Intensity | |
| (2θ, °) | (I/Iₒ₎ |
| 6.56 | 30 |
| 9.94 | 8 |
| 13.00 | 17 |
| 15.00 | 47 |
| 15.26 | 14 |
| 15.74 | 6 |
| 16.48 | 35 |
| 17.42 | 4 |
| 18.10 | 21 |
| 18.50 | 56 |
| 19.50 | 17 |
| 20.10 | 32 |
| 20.94 | 21 |
| 21.66 | 100 |
| 22.32 | 25 |
| 22.92 | 17 |
| 23.92 | 19 |
| 24.68 | 17 |
| 26.00 | 44 |
| 27.20 | 23 |
| 28.02 | 29 |
| 28.22 | 40 |
| 28.60 | 13 |

Wave numbers (cm⁻¹⁾ of infrared absorption spectra in potassium bromide are: 559, 641, 648, 702, 749, 765, 786, 807, 851, 872, 927, 949, 966, 975, 982, 1007, 1034, 1071, 1080, 1111, 1119, 1131, 1177, 1190, 1205, 1217, 1230, 1250, 1265, 1292, 1313, 1367, 1389, 1420, 1438, 1453, 1461, 1470, 1500, 1589, 1605, 1697, 2407, 2419, 2461, 2624, 2641, 2651, 2667, 2837, 2848, 2924, 2954, 2961, 2993, 3007, 3377, 3433 cm⁻¹.
Methods of making and using such compounds are described in U.S. Patent No. 6,140,321.

In one variation, the compound of a second therapy is a polymorphic crystal (A) of donepezil represented by the following formula: 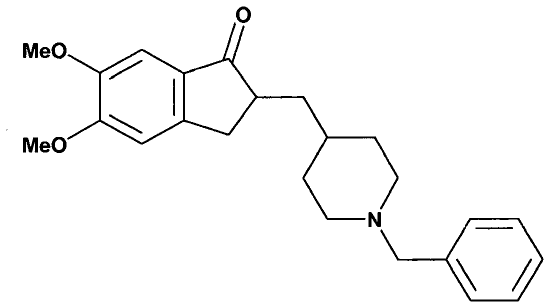 characterized by having peaks at the following diffraction angles (2θ) in its powder X-ray diffraction pattern;

| Diffraction angle (2θ, °) | Intensity (I/Iₒ) |
|---|---|
| 7.68 | 8 |
| 8.52 | 4 |
| 8.80 | 7 |
| 10.20 | 8 |
| 10.64 | 8 |
| 11.60 | 5 |
| 12.86 | 16 |
| 14.80 | 12 |
| 15.34 | 30 |
| 15.82 | 8 |
| 16.34 | 10 |
| 16.96 | 22 |
| 17.66 | 100 |
| 19.26 | 24 |
| 20.08 | 29 |
| 20.46 | 17 |
| 20.82 | 35 |
| 21.46 | 17 |
| 21.76 | 17 |
| 22.14 | 21 |
| 22.60 | 21 |
| 23.38 | 20 |
| 24.28 | 14 |
| 24.66 | 19 |
| 25.78 | 16 |

In one variation, the compound of a second therapy is a polymorphic crystal (B) of donepezil. The polymorphic crystal (B) of donepezil is characterized by having peaks at the following diffraction angles (2θ) in its powder X-ray diffraction pattern;

| Diffraction angle (2θ, °) | Intensity (I/Iₒ) |
|---|---|
| 5.82 | 79 |
| 11.28 | 8 |
| 11.46 | 26 |
| 11.58 | 60 |
| 11.86 | 5 |
| 12.04 | 10 |
| 12.30 | 36 |
| 13.02 | 17 |
| 13.30 | 38 |
| 13.66 | 7 |
| 13.88 | 11 |
| 14.40 | 6 |
| 15.34 | 8 |
| 15.46 | 10 |
| 16.46 | 9 |
| 16.60 | 14 |
| 16.74 | 13 |
| 18.00 | 11 |
| 18.30 | 8 |
| 18.56 | 9 |
| 19.34 | 12 |
| 19.48 | 26 |
| 19.70 | 42 |
| 20.24 | 9 |
| 20.76 | 15 |
| 21.34 | 14 |
| 21.58 | 24 |
| 21.72 | 33 |
| 21.90 | 100 |
| 22.76 | 13 |
| 22.90 | 16 |
| 23.22 | 26 |
| 23.48 | 35 |
| 23.82 | 14 |
| 24.04 | 17 |
| 24.22 | 38 |
| 24.52 | 8 |
| 24.66 | 12 |
| 25.60 | 20 |
| 28.04 | 7 |

In one variation, the compound of a second therapy is a polymorphic crystal (C) of donepezil. The polymorphic crystal polymorphic crystal (C) of donepezil is characterized by having peaks at the following diffraction angles (20θ) in its powder X-ray diffraction pattern:

| Diffraction angle (2θ, °) | Intensity (I/Iₒ) |
|---|---|
| 7.42 | 3 |
| 7.56 | 4 |
| 9.60 | 4 |
| 9.74 | 10 |
| 9.82 | 15 |
| 9.94 | 23 |
| 11.46 | 4 |
| 11.58 | 6 |
| 11.68 | 9 |
| 13.78 | 8 |
| 13.90 | 17 |
| 14.08 | 29 |
| 14.78 | 14 |
| 14.94 | 25 |
| 17.00 | 100 |
| 17.18 | 48 |
| 18.12 | 10 |
| 18.22 | 10 |
| 18.44 | 8 |
| 18.60 | 12 |
| 18.84 | 19 |
| 18.98 | 17 |
| 19.12 | 17 |
| 19.76 | 17 |
| 20.30 | 9 |
| 20.86 | 13 |
| 21.00 | 18 |
| 21.14 | 25 |
| 21.50 | 48 |
| 23.44 | 26 |
| 23.92 | 17 |
| 24.20 | 10 |
| 26.22 | 13 |
| 26.54 | 25 |
| 27.74 | 7 |
| 28.80 | 7 |

Methods of making and using such polymorphic crystals are described in U.S. Patent No. 6,245,911.

### Acetylcholine receptor agonists

Compounds for use in the systems, methods, combination therapies and kits described herein may include acetylcholine receptor agonists, such as neuronal nicotinic acetylcholine receptor agonists. Since the cholinergic system is impaired in Alzheimer's disease patients, acetylcholine receptor agonists are useful for improving symptoms of Alzheimer's disease. An exemplary agonist is TC-1734 (Targacept), which is an orally active neuronal nicotinic acetylcholine receptor modulator (agonist) with antidepressant, neuroprotective and long-lasting cognitive effects. This compound has high selectivity for neuronal nicotinic receptors. Microdialysis studies indicate that TC-1734 enhances the release of acetylcholine from the cortex.

### NMDA receptor antagonists

The second therapy herein may include use of NMDA receptor antagonists, which reduce or inhibit an activity of an NMDA receptor. Alzheimer's disease is associated with an excitotoxic effect of neuromediatory excitatory amino acids such as glutamate and aspartate (Excitatory Amino Acids and Drug Research, Ed. by M. R. Szewczak N. I. Hrib Alan R. Liss, Inc., New York, 1989, p.380; The NMDA Receptor. Eds. Watkins & Collingridge G., 1989, IRL Press). According to this mechanism, hyperexcitation of neurons due to the prolonged activation of their N-methyl-D-aspartate (NMDA) receptors with glutamate results in an excessive entry of potassium ions into the cell. The increase in potassium ions initiates a number of pathological metabolic processes, finally causing the death of nerve cells. (Mattson, Neuron, 1990, v.2, p.105, Mill S. Kater, Neuron, 1990, v.2, p.149; Saitch et al., Lab Suvest., 1991, v.64, p.596). β-amyloid significantly enhances the excitotoxic effect of glutamate through the NMDA-receptor system (Koh et al., Brain Res., 1990, v.533, p.315; Mattson et al., J. Neurosci., 1992, v. 12, p.376). As a result, the glutamate mediator at concentrations that are nontoxic under normal conditions becomes toxic for neurons under conditions of developing β-amyloid and causes their death. An exemplary and non-limiting list of NMDA receptor antagonists includes Memantine (Namenda® sold by Forest, Axura® sold by Merz, Akatinol® sold by Merz, Ebixa® sold by Lundbeck), Neramexane (Forest Labs), Amantadine, AP5 (2-amino-5-phosphonopentanoate, APV), Dextrorphan, Ketamine, MK-801 (dizocilpine), Phencyclidine, Riluzole and 7-chlorokynurenate. The structure of Neramexane is distinct from that ofNamenda but they are pharmacologically equivalent.

In one variation, the second therapy comprises an NMDA receptor antagonist of the formula (V): 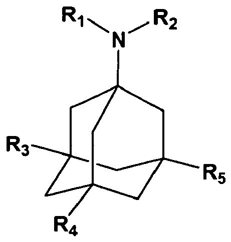 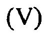 wherein R₁ and R₂ are identical or different, representing hydrogen or a straight or branched alkyl group of 1 to 6 C atoms or, in conjunction with N, a heterocyclic group with 5 or 6 ring C atoms; wherein R₃ and R₄ are identical or different, being selected from hydrogen, a straight or branched alkyl group of 1 to 6 C atoms, a cycloalkyl group with 5 or 6 C atoms, and phenyl; and wherein R₅ is hydrogen or a straight or branched C₁ -C₆ alkyl group, or a pharmaceutically-acceptable salt thereof, is disclosed. With reference to formula (V), branched or straight C₁ -C₆ alkyl groups representatively include methyl, ethyl, iso- and n-propyl, n-, iso- and t-butyl, n-pentyl, n-hexyl, and the isomers thereof. In one variation R₁, R₂ and R₅ are hydrogen. In one variation R₁, R₂ and R₅ are hydrogen, and R₃ and R₄ are methyl. In one variation R₁, R₂ and R₅ are hydrogen, and R₃ and R₄ are ethyl. In one variation R₁, R₂, R₄ and R₅ are hydrogen, and R₃ is ethyl, isopropyl, or cyclohexyl. In one variation R₂ and R₅ are hydrogen. In one variation R₃ and R₄ are methyl, R₂ and R₅ are hydrogen and R₁ is methyl or ethyl. In one variation R₁ and R₂ are hydrogen. In one variation R₁ and R₂ are hydrogen, R₃ is ethyl, and R₅ and R₄ are methyl. In one variation the second therapy comprises a compound selected from the group consisting of: 1-amino adamantine, 1-amino-3-phenyl adamantane , 1-amino-methyl-adamantane, 1-amino-3,5-dimethyl adamantane, 1-amino-3-ethyl adamantane, 1-amino-3-isopropyl adamantane, 1-amino-3-n-butyl adamantine, 1-amino-3,5-diethyl adamantane, 1-amino-3,5-diisopropyl adamantine, 1-amino-3,5-di-n-butyl adamantine, 1-amino-3-methyl-5-ethyl adamantine, I-N-methylamino-3,5-dimethyl adamantane, I-N-ethylamino-3,5-dimethyl adamantane, 1-N-isopropyl-amino-3,5-dimethyl adamantine, 1-N,N-dimethyl-amino-3,5-dimethyl adamantine, 1-N-methyl-N-isopropyl-amino-3-methyl-5-ethyl adamantine, 1-amino-3-butyl-5-phenyl adamantine, 1-amino-3-pentyl adamantine, 1-amino-3,5-dipentyl adamantine, 1-amino-3-pentyl-5-hexyl adamantine, 1-amino-3-pentyl-5-cyclohexyl adamantine, 1-amino-3-pentyl-5-phenyl adamantine, 1-amino-3-hexyl adamantine, 1-amino-3,5-dihexyl adamantine, 1-amino-3-hexyl-5-cyclohexyl adamantine, 1-amino-3-hexyl-5-phenyl adamantine, 1-amino-3-cyclohexyl adamantane, 1-amino-3,5-dicyclohexyl adamantine, 1-amino-3-cyclohexyl-5-phenyl adamantine, 1-amino-3,5-diphenyl adamantine, 1-amino-3,5,7-trimethyl adamantine, 1-amino-3,5-dimethyl-7-ethyl adamantane, 1-amino-3,5-diethyl-7-methyl adamantine, 1-N-pyrrolidino and I-N-piperidine derivatives, 1-amino-3-methyl-5-propyl adamantine, 1-amino-3-methyl-5-butyl adamantine, 1-amino-3-methyl-5-pentyl adamantine, 1-amino-3-methyl-5-hexyl adamantine, 1-amino-3-methyl-5-cyclohexyl adamantine, 1-amino-3-methyl-5-phenyl adamantine, 1-amino-3-ethyl-5-propyl adamantine, 1-amino-3-ethyl-5-butyl adamantine, 1-amino-3-ethyl-5-pentyl adamantine, 1-amino-3-ethyl-5-hexyl adamantine, 1-amino-3-ethyl-5-cyclohexyl adamantine, 1-amino-3-ethyl-5-phenyl adamantine, I-amino-3-propyl-5-butyl adamantine, 1-amino-3-propyl-5-pentyl adamantine, 1-amino-3-propyl-5-hexyl adamantine, 1-amino-3-propyl-5-cyclohexyl adamantine, 1-amino-3-propyl-5-phenyl adamantine, 1-amino-3-butyl-5-pentyl adamantine, 1-amino-3-butyl-5-hexyl adamantine, 1-amino-3-butyl-5-cyclohexyl adamantine, and their N-methyl, N,N-dimethyl, N-ethyl, N-propyl derivatives and their acid addition compounds. In one variation R₁ and R₂ are hydrogen such as, for example, 1-amino-3-ethyl-5,7-dimethyl adamantane, and compounds wherein R₁, R₂, R₄ and R₅ are hydrogen such as, for example, 1-amino-3-cyclohexyl adamantane and 1-amino-3-ethyl adamantane. In one variation R₁, R₂ and R₅ are hydrogen such as, for example, 1-amino-3-methyl-5-propyl or 5-butyl adamantane, 1-amino-3-methyl-5-hexyl or cyclohexyl adamantane, or 1-amino-3-methyl-5-phenyl adamantane. In one variation the compound is selected from the group of consisting of: 1-amino-3,5-dimethyl adamantane, 1-amino-3,5-diethyl adamantane, i.e., compounds wherein R₁, R₂ and R₅ are hydrogen, and compounds wherein R₁ and R₅ are hydrogen, R₂ is methyl or ethyl, and R₃ and R₄ are methyl such as, for example, 1-N-methylamino-3,5-dimethyl adamantane and 1-N-ethylamino-3,5-dimethyl adamantane. In one variation the derivatives may be used as such or in the form of their pharmaceutically-acceptable acid addition salts including, for example, the hydrochlorides, hydrobromides, sulfates, acetates, succinates or tartrates, or their acid addition salts with fumaric, maleic, citric, or phosphoric acids. Methods of making and using such compounds are described in U.S. Patent No. 5,061,703.

In one variation, the second therapy comprises an NMDA receptor antagonist of the formula (T): 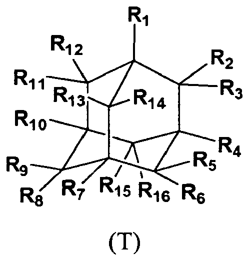 or a physiologically acceptable salt thereof wherein R₁ includes an amino group, and R₂ - R₁₇ are independently H or a short chain aliphatic group including 1-5 carbons, and R₄ and R₁₀ (independently) may also be a halogen (particularly fluorine, chlorine or bromine) or an acyl group. In one variation R₁ is NH₂, and the compound is preferably amantadine. In one variation R₄ is a methyl group. In one variation R₄ is a methyl group and R₁ is NH₂. In one variation R₁₀ is a methyl group. In one variation R₁₀ is a methyl group and R₁ is NH₂. In one variation R₄ and R₁₀ are methyl groups. In one variation R₄ and R₁₀ are methyl groups, and R₁ is NH₂. In one variation the said compound is memantine. In one variation R₁ is: 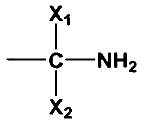 wherein X₁ and X₂ are independently H or a short chain aliphatic group including between 1-5 carbons. In one variation X₁ and X₂ are H and CH₃, respectively, or wherein X₁ and X₂ are CH₃ and H, respectively. In one variation the compound is rimantadine. In one variation R₁ is: 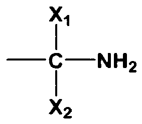 wherein X₁ and X₂ are independently H or a short chain aliphatic group including between 1-5 carbons, and R₄ is a methyl group. In one variation R₁ is: 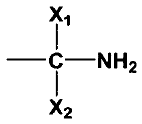 wherein X₁ and X₂ are independently H or a short chain aliphatic group including between 1-5 carbons, and R₁₀ is a methyl group. In one variation R₁ is: 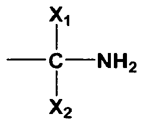 wherein X₁ and X₂ are independently H or a short chain aliphatic group including between 1-5 carbons, and R₄ and R₁₀ are methyl groups. Methods of making and using such compounds are described in U.S. Patent No. 5,614,560.

### Inhibitors of amyloid Aβ peptide or amyloid plaque

The second therapy may include use of inhibitors of amyloid Aβ peptide or amyloid plaque, such as a compound that reduces or eliminates the formation of β-amyloid precursor protein (β-APP), reduces or eliminates the formation of Aβ42, reduces or eliminates an activity of amyloid Aβ peptide or amyloid plaque, reduces or eliminates an interaction of a metal with a protein involved in Aβ oligomer formation, reduces or eliminates the formation of amyloid plaques (*e.g*., amyloid plaques in the brain), increases or promotes the clearance of amyloid plaques (*e.g*., amyloid plaques in the brain), binds to amyloid plaques (*e.g*., amyloid plaques in the brain), stimulates or increases an immune response against β-amyloid peptide, reduces or eliminates the amyloid cascade and/or reduces or eliminates Aβ peptide-induced toxicity or cell death. Reducing the amount of amyloid plaque is expected to improve, stabilize, eliminate, delay, or prevent Alzheimer's disease. In some embodiments, the compound reduces the amount of β-APP, Aβ42, amyloid plaque (*e.g.*, amyloid plaque in the brain), Aβ peptide-induced toxicity or cell death by at least or about 10%, 20%, 30%, 40%, 50%, 60%, 70%, 80%, 90%, 95% or 100% as compared to the corresponding amount in the same subject prior to treatment or compared to the corresponding amount in other subjects not receiving the combination therapy. An exemplary inhibitor is 3-amino-1-propanesulfonic acid (Tramiprosate, Alzhemed™) by Neurochem (Gervais et al., "Targeting soluble Abeta peptide with Tramiprosate for the treatment of brain amyloidosis," Neurobiol Aging, May 1, 2006). Alzhemed™ binds to soluble amyloid β (Aβ) peptide and interferes with the amyloid cascade. Posiphen (Axonyx) Posiphen™ reduces the formation of the β-amyloid precursor protein (β-APP) and amyloid and thus may inhibit Alzheimer's disease progression. Posiphen™ is the positive isomer of Phenserine. As such, it appears to affect the messenger RNA of β-APP as well as inhibit β-secretase, whereby neurotoxic levels of β-amyloid in preclinical animal models are reduced. Flurizan (Myriad) is a selective amyloid lowering agent (SALA) that reduces levels of the toxic peptide amyloid beta 42 (Aβ42) in cultured human cells and in animal models. Aβ42 is the primary initiator of neurotoxicity and amyloid plaque development in the brains of Alzheimer's disease patients. Kiacta or Fibrillex (NC-503, Eprodisate disodium, sodium1,3-propanedisulfonate, 1,3-propanedisulphonic acid, 1,3-PDS) is an orally active amyloid precursor protein antagonist that inhibits amyloid deposition. PBT-2 (Prana) reportedly reduces a pathological interaction of metals and proteins leading to toxic Aβ oligomer formation in the brain that is characteristic of patients with Alzheimer's disease. Memryte (leuprolide) (Voyager) appears to dramatically and significantly reduced the concentrations of brain Aβ42 in mice. This result is consistent with the hypothesis that abnormal gonadotropin levels also contribute to β-amyloid production. In particular, leuprolide is a synthetic analog of gonadotropin releasing hormone acting mainly on the pituitary gland in humans. Continuous treatment produces initial stimulation of FSH and LH (3-4days), then suppression, with reduction of gonadal hormones to castrate or postmenopausal levels (2-4 months). In males, the net effect is a reduction of testosterone to castration levels within two to four weeks. In females, both ovarian estrogen and androgen synthesis are inhibited.

AN-1792 (Elan/Wyeth) is a synthetic form of the β-amyloid peptide. Scientists have theorized that immunotherapy with the β-amyloid peptide may stimulate an immune response against the peptide that would, in turn, elicit clearance of β-amyloid peptide and plaques in the brains of those affected by Alzheimer's disease (active immunization). AAB-001 (Elan/Wyeth) is a humanized monoclonal antibody that binds to and clears β-amyloid peptide. This antibody to β-amyloid is administered to the patient so that the patient is not required to mount his own individual immune response to β-amyloid. Thus, this approach may eliminate the need for the patient to mount an immune response to β-amyloid. ACC-001 (Elan/Wyeth) is a β-amyloid related active immunization approach now in Phase I clinical trials. This approach is intended to induce a highly specific antibody response to β-amyloid. The goal is to clear β-amyloid while minimizing side effects such as inflammation of the central nervous system.

### cGMP-specific phosphodiesterase type 5 (PDE5) inhibitors

The second therapy may include use of cGMP-specific phosphodiesterase type 5 (PDE5) inhibitors, which reduce or inhibit an activity of a PDE5, such as the hydrolysis of cGMP. Because cyclic GMP may have neuroprotective effects, PDE5 inhibitors are expected to improve, stabilize, eliminate, delay, or prevent Alzheimer's disease. In some embodiments, the PDE5 inhibitor reduces the activity of PDE5 by at least or about 2, 5, 10, 100, 500, 1000, 2000, 3000, 40000-fold or more than the activity of PDE3, which is involved in control of cardiac contractibility, or PDE6, which is involved in the phototransduction pathway of the retina. Exemplary inhibitors are 1-[[3-(6,7-dihydro-1-methyl-7-oxo-3-propyl-1*H*pyrazolo[4,3-*d*]pyrimidin-5-yl)-4-ethoxyphenyl]sulfonyl]-4-methylpiperazine citrate (silednafil, Viagra, sold by Pfizer), (6R-trans)-6-(1,3-benzodioxol-5-yl)- 2,3,6,7,12,12a-hexahydro-2-methyl-pyrazino [1',2':1,6] pyrido[3,4-b]indole-1,4-dione, (tadalafil, Cialis, sold by LillyICOS),
Levitra (vardenafil, sold by Bayer Pharmaceutical and Glaxo-Smith-Kline-Beecham/ Schering Plough) and zaprinast (Nakamizo et al., "Phosphodiesterase inhibitors are neuroprotective to cultured spinal motor neurons," J. Neurosci. Res., 71 (4):485-95, Feb, 15, 2003). Viagra is approximately 4,000-fold as effective against PDE5 compared to PDE3 and 10-fold as effective against PDE5 compared to PDE6.

### Monoamine oxidase inhibitors

The second therapy may include use of monoamine oxidase inhibitors, such as compounds that inhibit the deamination of a monoamine neurotransmitter by a monoamine oxidase. An exemplary monoamine oxidase inhibitor is 5-(Nmethyl-N-propargyaminomethyl)-8-hydroxyquinoline, also referred to as M30. This compound is a multifunctional MAO-B inhibitor, neuroprotective, iron chelator (Zheng et al., "Novel multifunctional neuroprotective iron chelator-monoamine oxidase inhibitor drugs for neurodegenerative diseases: in vitro studies on antioxidant activity, prevention of lipid peroxide formation and monoamine oxidase inhibition. J Neurochem., 2005 Oct;95(1):68-78). Other exemplary monoamine oxidase inhibitors include isocarboxazid (Marplan), moclobemide (Aurorix, Manerix, Moclodura®), phenelzine (Nardil), tranylcypromine (Pamate), selegiline (Selegiline, Eldepryl), emsam, nialamide, iproniazid (Marsilid, Iprozid, Ipronid, Rivivol, Propilniazida), iproclozide and toloxatone. Many tryptamines, such as harmine, AMT, 5-MeO-DMT, and 5-MeO-AMT, have monoamine oxidase inhibitors properties. Ladostigil (TEVA, TV3326) produces brain-selective MAO-inhibition.

### Vascular endothelial cell growth factors

The second therapy may include use of vascular endothelial cell growth factor (VEGF) or a fragment thereof. Because VEGF may have neuroprotective effects, VEGF compounds are expected to improve, stabilize, eliminate, delay, or prevent Alzheimer's disease. Exemplary VEGF molecules include VEGF121, VEGF145, VEGF165, VEGF189, VEGF206, other gene isoforms and fragments thereof (Sun FY, Guo X. "Molecular and cellular mechanisms of neuroprotection by vascular endothelial growth factor," J Neurosci Res., 2005 Jan 1-15;79(1-2):180-4). In some embodiments, the VEGF fragment contains at least 25, 50, 75, 100, 150 or 200 contiguous amino acids from a full-length VEGF protein and has at least 5%, 10%, 20%, 30%, 40%, 50%, 60%, 70%, 80%, 90% or 100% of an activity of a corresponding full-length VEGF protein.

### Trophic growth factors

The second therapy may include use of trophic growth factors (*e.g.*,. IGF-1, FGF, NGF, BDNF, GCS-F and/or GMCS-F) and compounds that mimic their effect. GCS-F and GMCS-F stimulate new neuron growth. Because trophic growth factors may stimulate cell growth, they are expected to improve, stabilize, eliminate, delay, or prevent Alzheimer's disease. The combination of hydrogenated pyrido [4,3-b] indole such as dimebon and a trophic growth factor may reduce the apoptosis rate that is seen with new cell growth stimulation. An exemplary compound that mimics the effects of nerve growth factor is Xaliproden (Sanofi-Aventis) [SR 57746A, xaliprodene; Xaprila].

### Hypoxia inducible factor activators and HIF prolyl 4-hydroxylase inhibitors

The second therapy may include compounds that activate hypoxia inducible factor (HIF) and/or inhibit HIF prolyl 4-hydroxylases. Because hypoxia inducible factor activators and HIF prolyl 4-hydroxylase inhibitors may have neuroprotective effects, these compounds are expected to improve, stabilize, eliminate, delay, or prevent Alzheimer's disease. (Siddiq et al., "Hypoxia-inducible factor prolyl 4-hydroxylase inhibition. A target for neuroprotection in the central nervous system," J Biol Chem., 2005 Dec 16;280(50):41732-43. Epub 2005 Oct 13.)

### Anti-apoptotic or neuroprotective compounds

The second therapy may include use of anti-apoptotic compounds or neuroprotective compounds. Ladostigil (TEVA, TV3326) has neuroprotective activity.

### Activity-dependent neuroprotective protein agonists and analogs

The second therapy may include use of activity-dependent neuroprotective protein (ADNP) agonists and analogs. ADNP has been identified as a glial derived protein that has neuroprotective activity. Because ADNP peptide agonists and analogs have neuroprotective effects, they are expected to improve, stabilize, eliminate, delay, or prevent Alzheimer's disease. An exemplary ADNP analog is AL-108 (Allon), which is an intranasally formulated eight amino acid neuroprotective peptide analog of ADNP. AL-108 protects neurons against numerous toxins and cellular stresses including Alzheimer's disease neurotoxin (β-amyloid peptide), excitotoxicity (N-methyl-D-aspartate), the toxic envelope protein of HIV (gp120), electrical blockade (tetrodotoxin), oxidative stress (hydrogen peroxide), dopamine toxicity, decreased glutathione and tumor necrosis factor (TNF)-associated toxicity. In astrocytes, a cell population that constitutes a large and important proportion of the brain matter, AL-108 protects against death associated with microtubule dysfunction. AL-108 binds to tubulin and promotes assembly of microtubules, which comprise the key elements of the cellular skeleton in the nervous system.

### Activity-dependent neurotrophic factor agonists and analogs

The second therapy may include use of activity-dependent neurotrophic factor (ADNF) agonists and analogs. ADNF has been identified as a glial derived factor that has neuroprotective activity. Because ADNF peptide agonists and analogs have neuroprotective effects, they are expected to improve, stabilize, eliminate, delay, or prevent Alzheimer's disease. An exemplary ADNF peptide agonist is AL-208 (Allon), which is a nine amino acid peptide, Ser-Ala-Leu-Leu-Arg-Ser-Ile-Pro-Ala (SALLRSIPA), with neuroprotective activity.

### Activators of the AMPA-type glutamate receptor

The second therapy may include use of activators/positive modulators of the AMPA-type glutamate receptor. These compounds are expected to improve, stabilize, eliminate, delay, or prevent Alzheimer's disease. CX717 (Cortex) and CX516 (Cortex) are exemplary positive modulators of the AMPA-type glutamate receptor.

### Serotonin 5-HT1A receptor agonists

The second therapy may include use of serotonin 5-HT1A receptor agonists. These compounds are expected to improve, stabilize, eliminate, delay, or prevent Alzheimer's disease. Xaliproden (Sanofi-Aventis) [SR 57746A, xaliprodene; Xaprila] is an exemplary serotonin 5-HT1A receptor agonist, and it also mimics the effects of nerve growth factor.

### Serotonin 1A receptor antagonists

The second therapy may include use of serotonin 1A receptor antagonists. These compounds are expected to improve, stabilize, eliminate, delay, or prevent Alzheimer's disease. Lecozotan (SRA-333, Wyeth) is an exemplary selective serotonin 1A receptor antagonist that enhances the stimulated release of glutamate and acetylcholine in the hippocampus and possesses cognitive-enhancing properties.

### Nicotinic alpha-7 receptor agonists

The second therapy may include use of agonists of the nicotinic alpha-7 receptor. These compounds are expected to improve, stabilize, eliminate, delay, or prevent Alzheimer's disease. An exemplary compound includes MEM 3454 (Memory Pharma), which is a partial agonist of the nicotinic alpha-7 receptor.

### Neuronal L-type calcium channel modulators

The second therapy may include use of neuronal L-type calcium channel modulators. These compounds are expected to improve, stabilize, eliminate, delay, or prevent Alzheimer's disease. An exemplary compound includes MEM 1003 (Memory Pharma).

### 5-HT4 receptor agonists

The second therapy may include use of 5-HT4 agonists. These compounds are expected to improve, stabilize, eliminate, delay, or prevent Alzheimer's disease. An exemplary compound includes PRX-03140 (Predix), which is a highly selective, small-molecule agonist of a specific GPCR known as 5-HT4 that is useful for the treatment of Alzheimer's disease.

### Anti-inflammatory agents

The second therapy may include use of anti-inflammatory agents. These compounds are expected to improve, stabilize, eliminate, delay, or prevent Alzheimer's disease. An exemplary compound includes VP-025 (Vasogen), which is a bilayered phospholipid microparticle that interacts with macrophages and other cells of the immune system, eliciting an anti-inflammatory response.

### Administration, formulation, and dosing of compounds

Unless clearly indicated otherwise, the compounds in a combination therapy may be administered to the individual by any available dosage form. In one variation, one or more compounds in a combination therapy are administered to the individual as a conventional immediate release dosage form. In one variation, one or more compounds in a combination therapy are administered to the individual as a sustained release form or part of a sustained release system, such as a system capable of sustaining the rate of delivery of one or more compounds to an individual for a desired duration, which may be an extended duration such as a duration that is longer than the time required for a corresponding immediate-release dosage form to release the same amount (*e.g*., by weight or by moles) of compound(s), and can be hours or days. A desired duration may be at least the drug elimination half life of one or more of the administered compounds and may be about any of, *e.g*., at least about 6 hours or at least about 12 hours or at least about 24 hours or at least about 30 hours or at least about 48 hours or at least about 72 hours or at least about 96 hours or at least about 120 hours or at least about 144 or more hours, and can be at least about one week, at least about 2 weeks, at least about 3 weeks, at least about 4 weeks, at least about 8 weeks, or at least about 16 weeks or more. In one variation, one or more compounds in a combination therapy are administered as a conventional immediate release dosage form and one or more other compounds in the combination therapy are administered as a sustained release form.

A compound in a combination therapy may be formulated for any available delivery route, whether immediate or sustained release, including an oral, mucosal (*e.g*., nasal, sublingual, vaginal, buccal or rectal), parenteral (*e.g*., intramuscular, subcutaneous, or intravenous), topical or transdermal delivery form. A compound may be formulated with suitable carriers to provide delivery forms, which may be but are not required to be sustained release forms, that include, but are not limited to: tablets, caplets, capsules (such as hard gelatin capsules and soft elastic gelatin capsules), cachets, troches, lozenges, gums, dispersions, suppositories, ointments, cataplasms (poultices), pastes, powders, dressings, creams, solutions, patches, aerosols (*e.g*., nasal spray or inhalers), gels, suspensions (*e.g*., aqueous or non-aqueous liquid suspensions, oil-in-water emulsions or water-in-oil liquid emulsions), solutions and elixirs. The same or different routes of administration and delivery forms may be used for the compounds in a combination therapy.

The combined administration of (i) a hydrogenated pyrido [4,3-b] indole compound which is 2,8-dimethyl-5-(2-(6-methyl-3-pyridyl)ethyl)-2,3,4,5-tetrahydro-1H-pyrido[4,3-b]indole or a pharmaceutically acceptable salt thereof and (ii) donepezil, or a pharmaceutically acceptable salt thereof may include, *e.g.*, coadministration or concurrent administration using separate formulations or a single pharmaceutical formulation or consecutive administration in either order. For some embodiments of concurrent administration, the administration of the hydrogenated pyrido [4,3-b] indole overlaps the administration of donepezil or pharmaceutically acceptable salt thereof. In other embodiments, the administration is non-concurrent. For example, in some embodiments, the administration of the hydrogenated pyrido [4,3-b] indole is terminated before donepezil or pharmaceutically acceptable salt thereof is administered. In some embodiments, the administration of donepezil or pharmaceutically acceptable salt thereof is terminated before the hydrogenated pyrido [4,3-b] indole is administered. For sequential administration, there is preferably a time period while both (or all) pharmaceutically active compounds simultaneously exert their biological activities. Thus, the hydrogenated pyrido [4,3-b] indole may be administered prior to, during, or following administration of the donepezil or pharmaceutically acceptable salt thereof. In various embodiments, the timing between at least one administration of the hydrogenated pyrido [4,3-b] indole and at least one administration of the donepezil or pharmaceutically acceptable salt thereof is about 1 month or less, about 2 weeks or less, about 1 week or less, about 3 days or less, about 1 day or less, about 12 hours or less, about 6 hours or less, about 4 hour less, or about 2 hours or less. In another embodiment, the hydrogenated pyrido [4,3-b] indole and donepezil (or pharmaceutically acceptable salt thereof) are administered concurrently to the patient in a single formulation or separate formulations.

The amount of each pharmaceutically active compound (*e.g.*, dimebon, Aricept, Exelon, Razadyne, or Namenda) in a delivery form of a combination therapy may be any effective amount, *e.g.*, which may be from about 10 ng to about 1,500 mg or more. Exemplary dosages for the hydrogenated pyrido [4,3-b] indole and donepezil (or pharmaceutically acceptable salt thereof) in a combination therapy are those presently used and may optionally be lowered due to the combined action (*e.g.*, additive or synergy effect) of the compounds in a combination therapy. Thus, the doses required for the hydrogenated pyrido [4,3-b] indole and the donepezil (or pharmaceutically acceptable salt thereof) in a combination therapy may (but not necessarily) be lower than what is normally required when each compound is used alone. Therefore, in some embodiments, a subtherapeutic amount of the hydrogenated pyrido [4,3-b] indole and donepezil (or pharmaceutically acceptable salt thereof) in a combination therapy are administered. "Subtherapeutic amount" or "subtherapeutic level" refer to an amount that is less than the therapeutic amount, that is, less than the amount normally used when the hydrogenated pyrido [4,3-b] indole and the donepezil (or pharmaceutically acceptable salt thereof) are used alone. The reduction may be reflected in terms of the amount administered at a given administration and/or the amount administered over a given period of time (reduced frequency). In some embodiments, enough of the donepezil (or pharmaceutically acceptable salt thereof) is administered so as to allow reduction of the normal dose of the hydrogenated pyrido [4,3-b] indole required to effect the same degree of treatment by at least about any of 5%, 10%, 20%, 30%, 50%, 60%, 70%, 80%, 90%, or more. In some embodiments, enough of the hydrogenated pyrido [4,3-b] indole is administered so as to allow reduction of the normal dose of the donepezil (or pharmaceutically acceptable salt thereof) required to effect the same degree of treatment by at least or about any of 5%, 10%, 20%, 30%, 50%, 60%, 70%, 80%, 90%, or more. In some embodiments, the dose of the hydrogenated pyrido [4,3-b] indole and the other pharmaceutically active compound (or pharmaceutically acceptable salt thereof) per administration is less than about any of 1%, 2%, 3%, 4%, 5%, 6%, 7%, 8%, 9%, 10%, 11%, 12%, 13%, 14%, 15%, 18%, 20%, 25%, 30%, 35%, 40%, 45%, or 50% of the maximum tolerated dose for the compound when it is administered alone. In one variation, a delivery form, such as a sustained release system, comprises less than about 30 mg of each pharmaceutically active compound.

In one variation, a delivery form, such as a single sustained release system capable of multi-day administration, comprises an amount of compound such that the daily dose of compound is less than about 30 mg of compound. A treatment regimen involving a dosage form of a compound in a combination therapy, whether immediate release or a sustained release system, may involve administering the compound to the individual in a dose of between about 0.1 and about 10 mg/kg of body weight, at least once a day and during the period of time required to achieve the therapeutic effect. In other variations, the daily dose (or other dosage frequency) of the hydrogenated pyrido[4,3-b]indole and of donepezil (or pharmaceutically acceptable salt thereof) as described herein is between about 0.1 and about 8 mg/kg; or between about 0.1 to about 6 mg/kg; or between about 0.1 and about 4 mg/kg; or between about 0.1 and about 2 mg/kg; or between about 0.1 and about 1 mg/kg; or between about 0.5 and about 10 mg/kg; or between about 1 and about 10 mg/kg; or between about 2 and about 10 mg/kg; or between about 4 to about 10 mg/kg; or between about 6 to about 10 mg/kg; or between about 8 to about 10 mg/kg; or between about 0.1 and about 5 mg/kg; or between about 0.1 and about 4 mg/kg; or between about 0.5 and about 5 mg/kg; or between about 1 and about 5 mg/kg; or between about 1 and about 4 mg/kg; or between about 2 and about 4 mg/kg; or between about 1 and about 3 mg/kg; or between about 1.5 and about 3 mg/kg; or between about 2 and about 3 mg/kg; or between about 0.01 and about 10 mg/kg; or between about 0.01 and 4 mg/kg; or between about 0.01 mg/kg and 2 mg/kg; or between about 0.05 and 10 mg/kg; or between about 0.05 and 8 mg/kg; or between about 0.05 and 4 mg/kg; or between about 0.05 and 4 mg/kg; or between about 0.05 and about 3 mg/kg; or between about 10 kg to about 50 kg; or between about 10 to about 100 mg/kg or between about 10 to about 250 mg/kg; or between about 50 to about 100 mg/kg or between about 50 and 200 mg/kg; or between about 100 and about 200 mg/kg or between about 200 and about 500 mg/kg; or a dosage over about 100 mg/kg; or a dosage over about 500 mg/kg. In some embodiments, a daily dosage of dimebon and Aricept is administered, such as a daily dosage of each administered compound that is less than about 0.1 mg/kg, which may include but is not limited to, a daily dosage of about 0.05 mg/kg.

In some embodiments (for both simultaneous and sequential administrations), the hydrogenated pyrido [4,3-b] indole and donepezil (or pharmaceutically acceptable salt thereof) are administered at a predetermined ratio. For example, in some embodiments, the ratio of the weight of the hydrogenated pyrido [4,3-b] indole to the weight of donepezil (or pharmaceutically acceptable salt thereof) is about 1 to 1. In some embodiments, the weight ratio may be between about 0.001 to about 1 and about 1000 to about 1, or between about 0.01 to about 1 and 100 to about 1. In some embodiments, the ratio by weight of the hydrogenated pyrido [4,3-b] indole to the weight of donepezil (or pharmaceutically acceptable salt thereof) is less than about any of 100:1, 50:1, 30:1, 10:1, 9:1, 8:1, 7:1, 6:1, 5:1, 4:1, 3:1, 2:1, and 1:1 In some embodiments, the ratio by weight of the hydrogenated pyrido [4,3-b] indole and donepezil (or pharmaceutically acceptable salt thereof) is more than about any of 1:1, 2:1, 3:1, 4:1, 5:1, 6:1, 7:1, 8:1, 9:1, 30:1, 50:1, 100:1. Other ratios are contemplated.

A hydrogenated pyrido[4,3-b]indole (*e.g.*, dimebon) and donepezil may be administered to an individual in accordance with an effective dosing regimen for a desired period of time or duration, such as at least about one month, at least about 2 months, at least about 3 months, at least about 6 months, or at least about 12 months or longer. In one variation, the compound is administered on a daily or intermittent schedule for the duration of the individual's life. The compounds in a combination therapy may be administered for the same or different durations.

The dosing frequency can be about a once weekly dosing. The dosing frequency can be about a once daily dosing. The dosing frequency can be more than about once weekly dosing. The dosing frequency can be less than three times a day dosing. The dosing frequency can be less than about three times a day dosing. The dosing frequency can be about three times a week dosing. The dosing frequency can be about a four times a week dosing. The dosing frequency can be about a two times a week dosing. The dosing frequency can be more than about once weekly dosing but less than about daily dosing. The dosing frequency can be about a once monthly dosing. The dosing frequency can be about a twice weekly dosing. The dosing frequency can be more than about once monthly dosing but less than about once weekly dosing. The dosing frequency can be intermittent (*e.g.*, once daily dosing for 7 days followed by no doses for 7 days, repeated for any 14 day time period, such as about 2 months, about 4 months, about 6 months or more). The dosing frequency can be continuous (*e.g.*, once weekly dosing for continuous weeks). Any of the dosing frequencies can employ any of the compounds described herein together with any of the dosages described herein, for example, the dosing frequency can be a once daily dosage of less than 0.1 mg/kg or less than about 0.05 mg/kg each of dimebon and donepezil or a pharmaceutically acceptable salt thereof (such as Aricept).

The same or different dosing frequencies can be used for the compounds in a combination therapy. When administered separately, the hydrogenated pyrido [4,3-b] indole and donepezil or pharmaceutically acceptable salt thereof can be administered at different dosing frequency or intervals. For example, the hydrogenated pyrido [4,3-b] indole can be administered weekly, while donepezil or pharmaceutically acceptable salt thereof can be administered more or less frequently.

The dosage or dosing frequency of the hydrogenated pyrido [4,3-b] indole and donepezil or pharmaceutically acceptable salt thereof may be adjusted over the course of the treatment, based on the judgment of the administering physician.

### Pharmaceutical formulations

One or several compounds described herein can be used in the preparation of a formulation, such as a pharmaceutical formulation, by combining the compound or compounds as an active ingredient with a pharmacologically acceptable carrier, which are known in the art. Depending on the therapeutic form of the system (*e.g.*, transdermal patch vs. oral tablet), the carrier may be in various forms. In addition, pharmaceutical preparations may contain preservatives, solubilizers, stabilizers, re-wetting agents, emulgators, sweeteners, dyes, adjusters, salts for the adjustment of osmotic pressure, buffers, coating agents or antioxidants. Preparations comprising a combination therapy may also contain other substances which have valuable therapeutic properties. Pharmaceutically active compound in a combination therapy can be prepared as part of the same or different formulations to be administered together or separately. Therapeutic forms may be represented by a usual standard dose and may be prepared by a known pharmaceutical method. Suitable doses of any of the coadministered compounds may optionally be lowered due to the combined action (*e.g.*, additive or synergistic effects) of the compounds. Suitable formulations can be found, *e.g.,* in Remington's Pharmaceutical Sciences, Mack Publishing Company, Philadelphia, PA, 20th ed. (2000), which is incorporated herein by reference.

In some embodiments, the amount of the first therapy, the second therapy, or the combined therapy in a pharmaceutical composition is an amount sufficient to increase the amount or activity of acetylcholine, reduce an activity of an acetylcholinesterase, increase an activity of an acetylcholine receptor, reduce an activity of an NMDA receptor, reduce an activity of an amyloid Aβ peptide, reduce the amount of amyloid plaque, reduce an activity of a PDE5 or PDE4, reduce an activity of a monoamine oxidase, increase an activity or amount of a VEGF protein, increase an activity or amount of a trophic growth factor, increase an activity of a HIF, reduce an activity of a HIF prolyl 4-hydroxylases, increase an activity or amount of an ADNP, increase an activity or amount of an ADNF, increase an activity of AMPA-type glutamate receptor, increase an activity of a serotonin 5-HT1A receptor, reduce an activity of a serotonin 1A receptor, increase an activity of a nicotinic alpha-7 receptor, modulate an activity of a neuronal L-type calcium channel, increase an activity of a 5-HT4 receptor, decrease the amount of inflammation and/or have a neuroprotective effect (*e.g*., inhibit cell death). In some embodiments, one or more of these activities changes by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. In some embodiments, the amount of the first therapy, the second therapy, or the combined therapy in a pharmaceutical composition is an amount sufficient to produce a desired therapeutic outcome (*e.g*., reducing the severity or duration of, stabilizing the severity of, or eliminating one or more symptoms of Alzheimer's disease). In various embodiments, the amount of the first therapy, the second therapy, or the combined therapy in a pharmaceutical composition is an amount sufficient to prevent or reduce the severity of one or more future symptoms of Alzheimer's disease when administered to an individual who is susceptible and/or who may develop Alzheimer's disease.

The first and second compounds of a combination therapy may be combined with a pharmaceutically acceptable carrier to produce a pharmaceutical composition. In certain embodiments, the pharmaceutical formulations encompass combination therapy dosage forms in which the first and second compounds of a combination therapy are present in a unit dosage form. As used herein, the term "unit dosage form" refers to a combination therapy formulation that contains a predetermined dose of a first compound (such as dimebon), and a predetermined dose of a second compound (such as donepezil). The first and second compounds of the combination therapy unit dosage form are present in amounts effective to treat AD.

### Kits

The invention further provides a product comprising: (a) a first therapy comprising 2,8-dimethyl-5-(2-(6-methyl-3-pyridyl)ethyl)-2,3,4,5-tetrahydro-1H-pyrido[4,3-b]indole, or pharmaceutically acceptable salt thereof, and (b) a second therapy comprising donepezil or pharmaceutically acceptable salt thereof;
as a combined preparation for simultaneous, separate or sequential use in the treatment, prevention, slowing the progression or delaying the onset and/or development of Alzheimer's disease.
The product may be provided as a kit. In one variation, the kit employs dimebon. The compound may be formulated in any acceptable form. The kits may be used for any one or more of the uses described herein, and, accordingly, may contain instructions for any one or more of the stated uses (*e.g*., treating and/or preventing and/or delaying the onset and/or the development of Alzheimer's disease).
In various embodiments, kit includes a compound that increases the amount or activity of acetylcholine (*e.g.*, an acetylcholinesterase inhibitor, a butyrylcholinesterase inhibitor or an acetylcholine receptor agonist), a NMDA receptor antagonist, an inhibitor of amyloid Aβ peptide or amyloid plaque, a PDE5 inhibitor, a PDE4 inhibitor, a monoamine oxidase inhibitor, a VEGF protein, a trophic growth factor, a HIF activator, a HIF prolyl 4-hydroxylase inhibitor, an anti-apoptotic compound, an ADNP agonist or analog, an ADNF agonist or analog, an activator of an AMPA-type glutamate receptor, a serotonin 5-HT1A receptor agonist, a serotonin 1A receptor antagonist, a nicotinic alpha-7 receptor agonist, a neuronal L-type calcium channel modulator, a 5-HT4 receptor agonist, and/or an anti-inflammatory agent. In some embodiments, the amount of the first therapy, the second therapy, or the combined therapy in a kit is an amount sufficient to increase the amount or activity of acetylcholine, reduce an activity of an acetylcholinesterase or a butyrylcholinesterase, increase an activity of an acetylcholine receptor, reduce an activity of an NMDA receptor, reduce an activity of an amyloid Aβ peptide, reduce the amount of amyloid plaque, reduce an activity of a PDE5 or PDE4, reduce an activity of a monoamine oxidase, increase an activity or amount of a VEGF protein, increase an activity or amount of a trophic growth factor, increase an activity of a HIF, reduce an activity of a HIF prolyl 4-hydroxylases, increase an activity or amount of an ADNP, increase an activity or amount of an ADNF, increase an activity of AMPA-type glutamate receptor, increase an activity of a serotonin 5-HT1A receptor, reduce an activity of a serotonin 1A receptor, increase an activity of a nicotinic alpha-7 receptor, modulate an activity of a neuronal L-type calcium channel, increase an activity of a 5-HT4 receptor, decrease the amount of inflammation and/or have a neuroprotective effect (*e.g.*, inhibit cell death). In some embodiments, one or more of these activities changes by at least or about 10%, 20%, 30%, 40%, 50%, 60%, 70%, 80%, 90%, 95% or 100% as compared to the corresponding activity in the same subject prior to treatment or compared to the corresponding activity in other subjects not receiving the combination therapy. In some embodiments, the amount of the first therapy, the second therapy, or the combined therapy in the product is an amount sufficient to produce a desired therapeutic outcome (*e.g.*, reducing the severity or duration of, stabilizing the severity of, or eliminating one or more symptoms of Alzheimer's disease). In various embodiments, the amount of the first therapy, the second therapy, or the combined therapy in the product is an amount sufficient to prevent or reduce the severity of one or more future symptoms of Alzheimer's disease when administered to an individual who is susceptible and/or who may develop Alzheimer's disease.

Kits generally comprise suitable packaging. The kits may comprise one or more containers comprising any compound described herein. Suitable packaging includes, but is not limited to, vials, bottles, jars, flexible packaging (*e.g*., plastic bags), and the like. Each component (if there is more than one component) can be packaged in separate containers or some components can be combined in one container where cross-reactivity and shelf life permit. Kits may optionally provide additional components such as buffers.

The kits may optionally include a set of instructions, generally written instructions, although electronic storage media (*e.g*., magnetic diskette or optical disk) containing instructions are also acceptable, relating to the use of component(s) of the methods relating to the present invention (*e.g*., treating, preventing and/or delaying the onset and/or the development of Alzheimer's disease). The instructions included with the kit generally include information as to the components and their administration to an individual, such as information regarding dosage, dosing schedule, and route of administration.

The containers may be unit dosage forms, bulk packages (*e.g*., multi-dose packages) or sub-unit doses. For example, kits may be provided that contain sufficient dosages of (i) the compound which is 2,8-dimethyl-5-(2-(6-methyl-3-pyridyl)ethyl)-2,3,4,5-tetrahydro-1H-pyrido[4,3-b]indole or a pharmaceutically acceptable salt thereof, and (ii) donepezil or a pharmaceutically acceptable salt thereof to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the compounds and instructions for use and be packaged in quantities sufficient for storage and use in pharmacies (*e.g*., hospital pharmacies and compounding pharmacies).

The following Examples are provided to illustrate the invention.

### EXAMPLES

### Example 1. Randomized, double-blinded, placebo-controlled Alzheimer's disease study using dimebon.

Dimebon, 2,8-dimethyl-5-(2-(6-methyl-3-pyridyl)-ethyl)-2,3,4, 5-tetrahydro-1H-pyrido(4,3-b)indol dihydrochloride, was used as a representative compound of hydrogenated pyrido (4,3-b) indoles and was found to improve cognition, function and behavior in human patients with Alzheimer's disease. 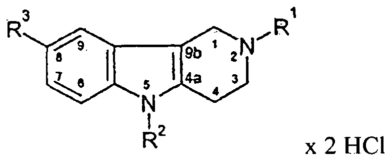 where R¹ and R³ are methyls, and R² is 2-(6-methyl-3-pyridyl)-ethyl.

In the study, 183 patients with mild to moderate Alzheimer's disease were randomized to dimebon (20 mg orally three times a day) or placebo for 6 months. Patients were evaluated with the ADAS-cog (primary endpoint), CIBIC-plus, MMSE, NPI and ADL at baseline, week 12 and week 26. The Alzheimer's Disease Assessment Scale - Cognitive Subscale (ADAS-cog) score assesses memory and cognition over time. The Mini Mental State Exam (MMSE) also assesses memory and cognition. The Alzheimer's Disease Cooperative Study-Clinical Global Impression of Change (ADCS-CGIC, also called CIBIC-plus) measures the patient's global status over time. It takes into account memory, cognition, behavior and motor disturbance. The Neuropsychiatric Inventory (NPI) measures the patients' behavior and psychiatric disturbance in 12 domains including delusions, hallucinations, agitation/aggression, depression/dysphoria, anxiety, elation/euphoria, apathy/indifference, disinhibitions, irritability/lability, motor disturbance, nighttime behaviors, and appetite/eating. An ADL inventory assesses the impact of cognitive impairment on activities of daily living. Eighty four percent of patients completed the trial (dimebon 87.6; placebo 81.9). All subjects enrolled were included in the intention-to-treat analysis. Thus, the analysis includes all randomized patients, even those who discontinued the study prior to study completion. Treatment with dimebon resulted in statistically-significant improvements in ADAS-cog, CIBIC-plus, MMSE, NPI and ADL scores relative to placebo at week 26. Scales used to evaluate dimebon are known by those of skill in the art and are described, *e.g*., by Delegarza, V. W., 2003, American Family Physician, 68:1365-1372 and Tariot, P.N. et al., 2000, Neurology, 54:2269-2276.

At week 26, the mean screening MMSE was 18.3 (SD 3.3). The mean drug-placebo differences included: ADAS-cog (4.0 units, p < 0.0001); CIBIC-plus (0.61 units, p<0.0001); MMSE (2.24 units, p < 0.0001); NPI (3.57 units, p = 0.006) and ADL (3.35 units, p = 0.0016). Treatment with dimebon also resulted in significant improvements in all 5 endpoints when the mean baseline scores were compare with the week 26 scores. Fewer dimebon-treated patients experienced serious adverse events than did placebo patients (2.2% vs. 7.4%). The most common adverse event in dimebon-treated patients was dry mouth (13.5%). All other gastrointestinal side effects combined occurred in <3% of patients. At week 52, the mean drug-placebo differences included: ADAS-cog (6.9 units, p < 0.0001); CIBIC-plus (0.80 units, p = 0.0058); MMSE (2.3 units, p < 0.0009); NPI (3.5 units, p = 0.04) and ADL (5.2 units, p = 0.004). Treatment with dimebon also resulted in improvements in all 5 endpoints when the mean baseline scores were compare with the week 52 scores. Fewer dimebon-treated patients experienced serious adverse events than did placebo patients (3.4 % vs. 11.7 %). The most common adverse event in dimebon-treated patients was dry mouth (18%). All other gastrointestinal side effects combined occurred in <3 % of patients.

Dimebon-treated patients were significantly improved compared to placebo patients on all five endpoints. In addition, dimebon-treated patients were significantly improved on all five endpoints at 6 months (*i.e*., week 26) and 12 months (*i.e*., week 52) as compared to mean baseline assessments at the beginning of the trial. Dimebon is a well-tolerated drug that improved cognition, function and behavior in patients with mild-moderate Alzheimer's disease.

### Example 2. Double-blind, placebo-controlled, randomized study to obtain extended safety, tolerability, and preliminary efficacy assessments of combination therapy with orally administered dimebon and donepezil for the treatment of Alzheimer's disease.

The following study is conducted in two parts. The first is a placebo-controlled, randomized, within-patient dose titration study of the safety, tolerability, and pharmacokinetics of orally administered dimebon in Alzheimer's disease ("AD") patients on the acetylcholinesterase inhibitor donepezil. The second is a double-blind, placebo-controlled, randomized study to obtain extended safety, tolerability, and preliminary efficacy assessments of orally-administered dimebon in AD patients on donepezil.

One objective of the first part of the study is to assess the safety and tolerability of orally-administered dimebon in patients with AD also taking a stable dosage of donepezil (marketed under the trade name Aricept^{®}). Additional objectives of the first part of the study are: (1) to assess the pharmacokinetics of orally-administered dimebon in patients with AD taking a stable dosage of donepezil; (2) to assess the pharmacokinetics of orally-administered donepezil at a dose of 10 mg per day, administered once daily to patients with AD during a within-patient dose titration of dimebon.

One objective of the second part of the study is to assess the safety and tolerability of orally-administered dimebon dosed for up to three months in patients with AD on a stable dosage of donepezil. An additional objective of the second part of the study is to estimate the extended term impact of dimebon co-administration on steady-state plasma donepezil concentrations. A further objective of the second part of the study is to obtain a preliminary assessment of the additional therapeutic benefits that orally-administered dimebon affords AD patients on a stable dosage of donepezil as determined by performance on the following assessments: (1) Alzheimer's Disease Assessment Scale, Cognitive Subscale ("ADAS-cog"); (2) Clinician's Interview-Based Impression of Change, plus input from the caregiver ("CIBIC-plus"); (3) Mini-Mental State Examination ("MMSE"); (4) Alzheimer's Disease Cooperative Study-Activities of Daily Living ("ADCS-ADL"); and (5) Neuropsychiatric inventory ("NPI").

### Part 1: Within-patient dose-titration study of the safety, tolerability, and pharmacokinetics of orally-administered dimebon in Alzheimer's disease patients on donepezil.

To assess the safety, tolerability, and pharmacokinetics of orally-administered dimebon, a phase 1 double-blind, placebo-controlled, randomized study is conducted on AD patients taking a stable dosage (10 mg once a day) of donepezil.

Study participants are selected after a screening visit at the Phase 1 unit at the study site. During the screening visits, informed written consent is obtained; a screening number is assigned; inclusion and exclusion criteria are reviewed; comprehensive AD assessment is performed using ADAS-cog, CIBIC-plus, MMSE, ADCS-ADL, and NPI; a medical history is taken (including history of caffeine, alcohol, and tobacco use); other medications being taken by the patient are recorded; vital signs are measured; weight, height, and BMI are determined; a physical examination is performed; a urine specimen is collected for drug testing; fasting blood and urinalysis specimens (overnight or for 8 hours) are collected; blood and plasma samples are collected and frozen on-site to serve as a baseline reference if needed; a 12-lead electrocardiogram is taken (after the subject rests supine for at least five minutes before recording); and instructions for evening dosing of donepezil and reporting of adverse events ("AEs") are provided.

Study participants are those meeting all of the following criteria: (1) age (50 years or older); (2) clinical diagnosis of AD according to the National Institute of Neurological and Communicative Disorders and Stroke-Alzheimer's Disease and Related Disorder Association's Criteria for probable AD or the Diagnostic and Statistical Manual of Mental Disorders, Fourth Edition, Text Revision's Criteria for AD; (3) on a 10 mg dose of donepezil for at least 60 days by Study Day 1; (4) on an evening dosing regimen for donepezil for at least 3 days prior to Study Day 1 and tolerating it well, including an absence of any gastrointestinal side effects; and (5) otherwise in general good health and ambulatory.

The following events may result in the removal of subjects from the study, either permanently, or until the etiology of the problem has been identified and resolved: (1) dimebon intolerance as assessed by the subject, the investigator, or the medical monitor; (2) seizure; (3) creatinine levels greater than 2 mg/dL; (4) liver function test (AST or ALT) showing levels greater than three times the ULN; (5) absolute neutrophil count of less than 1000/µL; or (6) platelet concentration of less than 100,000/ µL. Patients who discontinue the study for any reason are followed for the collection of safety data through the follow-up visit. If a subject refuses to return for any further visits, procedures for the follow-up visit are completed if possible. For subjects who refuse further clinic study visits, a documented telephone contact is attempted to review for AEs seven days after the last dose of study drug.

Twenty-one (21) patients are randomized to receive dimebon or a placebo in a 2:1 ratio (14 dimebon:7 placebo). Patients assigned to the placebo group are given matching placebo capsules to facilitate blinding. Dosage is titrated in weekly 2-fold increments within each patient over a three week period, starting at 2.5 mg three times a day (TID) and titrating up to a maximum dosage of 20 mg TID.

Within-patient dose-titration of dimebon is performed weekly until a patient experiences intolerable side effects or a maximum dosage of 20 mg TID is achieved. Throughout the study, safety and tolerability are assessed by monitoring adverse events ("AEs"), vital signs, 12-lead electrocardiograms (ECGs), and by conducting regular physical exams and laboratory studies. Pharmacokinetic ("PK") profiling of orally-administered dimebon is performed after 3 full days of dosing at each dosage administered.

The day before the study begins ("study day -1"), patients are given one 10 mg dose of donepezil in the evening. For the first seven days of the study ("study days 1 - 7"), patients are given dimebon or placebo at a dose of 2.5 mg three times a day ("TID") and one 10 mg dose of donepezil in the evening. For the second seven days of the study ("study days 8 - 14"), patients are given dimebon or placebo at a dose of 5 mg TID and one 10 mg dose of donepezil in the evening. For the third seven days of the study, ("study days 15 - 21 "), patients are given dimebon or placebo at a dose of 10 mg TID, and one 10 mg dose of donepezil in the evening. For the fourth and final seven days of the study ("study days 22-28"), patients are given dimebon or placebo at a dose of 20 mg TID, and one 10 mg dose of donepezil in the evening. Patients are dosed with a single capsule of dimebon at the appropriate dose strength or matching placebo.

Multiple blood samples for PK analysis are collected on study days 3, 10, 17, and 24. Single blood samples to measure trough plasma dimebon concentrations are collected on study days 4, 11, 18, and 25. Single blood samples are collected for genotyping of cytochrome P450 (CYP) isoforms, including one or more of CYP2D6, CYP2C9, and CYP2C19, on Study Day -1 to explore the potential relationship between CYP2D6 and CYP2C activity level and PK parameters if indicated. Patients are admitted to the Phase 1 unit on Study Day -1, the day before dimebon dosing begins.

For the first three days of each seven day dimebon dosing period (*i.e.*, study days 1-7, 8-14, 15-21, and 22-28), patients are admitted to the Phase 1 unit at the study site for inpatient clinical monitoring for the first three days of dosing (all of days 1 to 3, and the morning of day 4 for each seven day dosing period). On the fourth day of each seven day dosing period, patients are discharged to home on seizure precautions (no driving, no operation of heavy machinery, no proximity to bodies of water without being under observation, no sleeplessness, and no excessive alcohol consumption). Patients are administered dimebon or placebo plus donepezil on the same dosage schedule through each seven day dosing period. Patients are re-admitted to the Phase 1 unit at the study site on the seventh day of each dosing period, the day before the dose of dimebon or placebo is increased. Patients return to the Phase 1 unit at the study site on study day 28 to complete the last day of dosing. As an alternative to the schedule of 4 days inpatient and 3 days outpatient per seven day dosing period of dimebon or placebo, patients are admitted to stay in the Phase 1 unit at the study site for the entire 28 days of dimebon dosing. Minor deviations from scheduled time points during the study to accommodate simultaneously scheduled assessments will not be considered protocol violations or deviations as long as assessments are obtained as closely as possible to the scheduled time point.

Throughout the study, AD symptoms are assessed by spontaneous reporting of AEs and by evaluating patients response to non-leading questions immediately prior to taking vital signs and/or immediately prior to PK sampling. Furthermore, patients' vital signs, weight, urine output, and other physiological parameters are monitored throughout the study as necessary, including by conventional physical examination, electrocardiogram ("ECG") and the like. Pharmacokinetic profiling of orally-administered dimebon is performed on the first and the third days of TID (three times a day) dosing with dimebon at the specified doses for each seven day dosing period (*e.g.*, on study days 1 and 4 of the first dosing period). In addition, pre-dose (trough) plasma PK samples are drawn before the morning dose of dimebon on the fourth day of dosing in each seven day dosing period (*e.g*., on study days 4, 11, 18, and 25).

On the first day of dosing of each seven day dosing period (*i.e.,* on study days 1, 8, 15, and 22), PK plasma samples are collected before (within 10 minutes) the morning dosing of dimebon. On the third day of dosing (*i.e*., on study days 3, 10, 17, and 24) PK plasma samples are collected at the following time points: (1) before (within 10 minutes) administration of the morning dose of dimebon; (2) at 2, 3, 4, and 6 hours (±10 minutes) after the administration of the morning dose of dimebon; (3) at 2, 3, 4, and 6 hours (± 10 minutes) after the administration of the afternoon dose of dimebon; and (4) at 2, 3, and 4 hours (± 10 minutes) after administration of the evening dose of dimebon. On the fourth day of dosing of each seven day dosing period (*i.e.*, on study days 4, 11, 18, and 25), PK plasma samples are collected before (within 10 minutes) of administration of the morning dose of dimebon. Sampling for plasma concentrations of dimebon is also performed with blood draws occurring as part of the workup for AEs, where appropriate.

Plasma sampling for determination of baseline donepezil concentrations is performed on the day before initiation of dosing with dimebon (*i.e.,* on study day -1), with samples taken immediately before administration of the daily dose of donepezil (given at approximately 8:00 PM) and 4 hours after the dose. Plasma donepezil concentrations are measured again on study day 24, by which time steady-state levels of dimebon should have been reached for the highest tolerated dose of dimebon. Plasma samples for determining donepezil concentrations are taken on study day 24 on the same schedule as on study day -1. Sampling for plasma concentrations of donepezil will also be performed with blood draws occurring as part of the workup for AEs where appropriate.

Furthermore, investigation of the potential for dimebon to alter the cholinesterase inhibiting activity of donepezil is conducted if AEs consistent with a hypercholinergic state are encountered with a greater frequency or severity than expected from donepezil alone, or if other clinical findings suggest effects that could be mediated by altered acetylcholinesterase ("AChE") or butylcholinesterase ("BChE") inhibition. Blood samples for determination of AChE and BChE activity are collected on study days -1 and 24, with blood draws occurring as part of the workup for AEs as necessary. All samples will be processed and stored onsite until the end of the study, after which time appropriate samples are assayed for AChE and BChE. Samples are either shipped to a central laboratory for assay or destroyed within 2 months of the last patient visit.

The primary assessment for this study is measures of the safety and tolerability of dimebon when administered in conjunction with donepezil. The frequency and severity of AEs, laboratory abnormalities, and ECG changes in the dimebon group are compared to those reported in the placebo group.

An additional assessment for this study is PK characterization of orally-administered dimebon starting with a 2.5 mg TID dosage and escalating within-subject in increments of 2.5, 5, or 10 mg TID per week to a maximum dosage of 20 mg TID in AD patients on a stable dosage (10 mg QD) of donepezil. The specific PK parameters measured include (1) Cₘₐₓ (maximum observed plasma concentration of dimebon after a single dose); (2) tₘₐₓ (time Cₘₐₓ occurs); (3) AUC₀₋ₜ (area under the dimebon plasma concentration versus time curve from time zero to the last sampling time); (4) AUC_{0-∞} (area under the dimebon plasma concentration versus time curve from time zero to infinity calculated from AUC₀₋ₜ + (Cₜ/λ_{z}), where Cₜ is the last observed quantifiable concentration); (5) λ_{z} (apparent terminal elimination rate constant of dimebon); (6) t_{1/2} (apparent terminal half-life of dimebon, calculated from (loge 2)/λ_{z}); (7) CL/F (apparent total plasma clearance of dimebon, calculated from dose/AUC_{0-∞} where F is the oral bioavailability); and (8) V_{z}/F (apparent volume of distribution of dimebon, calculated as dose/(AUC_{0-∞} × λ_{z}), where F is the oral bioavailability). For dose-dependent pharmacokinetic parameter calculations (*i.e*., CL/F and V_{z}/F), the dose is corrected for free base content using a dihydrochloride salt/base ratio of 0.814.

Another assessment for this study is the characterization of changes in the PK characteristics of orally-administered donepezil (10 mg QD) while co-administering dimebon, starting with a 2.5 mg TID dosage and escalating within-subject in increments of 2.5, 5, or 10 mg TID per week to a maximum dosage of 20 mg TID in AD patients. The specific PK parameters measured are those as listed above for dimebon. At a minimum, trough donepezil concentrations from pre-dose PK sampling time points on study days -1 and 24 are measured. Additional PK sampling time points are analyzed for donepezil plasma concentration and additional PK parameters are calculated based on the clinical findings and dimebon PK findings.

Within one week after their last scheduled visit to the Phase 1 unit (study day 28), study participants, accompanied by their caregivers, are examined at their referring physician's offices for their first post-screening visit of the outpatient extension study, described in Part 2, below.

### Part 2: A double-blind, placebo-controlled, randomized study to obtain extended safety, tolerability, and preliminary efficacy assessments of orally-administered dimebon in Alzheimer's disease patients on donepezil.

To assess the safety and tolerability of orally-administered dimebon administered for up to three months, a phase 1 double-blind, placebo-controlled, randomized study is conducted on AD patients taking a stable dosage (10 mg once a day) of donezepil. The first visit of Part 2 of the study is made within seven days of the last visit to the Phase 1 unit for Part 1 of the study, and no later than study day 36 of Part 1. Study visits for Part 2 are made weekly. Patients are initially randomized to receive dimebon or placebo. All study participants also receive a 10 mg dose of donezepil once daily, in the evening.

For those initially receiving dimebon, dosing is at the maximum tolerated dose (≤20 mgTID) as determined in part 1, and is completed by the week 8 study visit for Part 2. Those patients are followed for week 9 and week 10 study visits to complete safety assessments and then discharged from the study. Patient vital signs, weight and the like are monitored at weekly study and follow-up visits by physical examination, ECG, and other standard clinical methods. PK parameters, including plasma concentrations of donezepil and dimebon, are monitored with blood and/or plasma samples taken as needed at weekly study visits. At weeks 4 and 8, a comprehensive AD assessment is performed using MMSE, ADAS-cog, CIBIC-plus, ADCS-ADL, and NPI.

Those initially receiving placebo are administered dimebon at 2.5 mg TID starting at the week 8 study visit. The dose of dimebon is titrated up weekly in 2-fold increments as described in Part 1, until the maximum individually tolerated dose is reached (≤20 mg TID) at week 12. Once those patients are at their maximum tolerated dose of dimebon, dosing continues according to study protocol. Patients receive dimebon at the maximum tolerable dose for eight weeks, and are monitored in weekly study visits until week 20. At week 20, dimebon dosing stops. Those patients are followed for two more weekly study visits to complete safety assessments, and then discharged from the study. Patient vital signs, weight and the like are monitored at the weekly study and follow-up visits by physical examination, ECG, and other standard clinical methods. PK parameters, including plasma concentrations of donezepil and dimebon, are monitored with blood and/or plasma samples taken as needed at weekly study visits. At weeks 16 *(i.e.,* 8 weeks of placebo + 4 weeks of within-patient dose-titration + 4 weeks of treatment at the maximum tolerated dose of dimebon plus donezepil) and 20 (*i.e.,* 8 weeks of placebo + 4 weeks of within-patient dose-titration + 8 weeks of treatment at the maximum tolerated dose of dimebon plus donezepil), a comprehensive AD assessment is performed using MMSE, ADAS-cog, CIBIC-plus, ADCS-ADL, and NPI.

After conclusion of the study, the frequency and severity of AEs, laboratory abnormalities, and ECG changes is compared to those reported for the placebo group. Also, the morning plasma concentrations of donezepil are evaluated to estimate the longer-term impact of dimebon co-administration on steady-state plasma concentrations of donezepil. Finally, changes in the ADAS-cog, CIBIC-plus, MMSE, ADCS-ADL, and NPI scores over time are assessed via comparison within treatment groups and between treatment groups to assess the efficacy of combination therapy in treating, preventing and/or delaying the onset and/or the development of Alzheimer's disease. Comparison of changes in scores on any or all of those assessments from the initial screening visit to scores from the week 8 study visit for the dimebon-treated group versus the placebo-treated group are particularly informative. Similarly, comparison of changes from scores on any or all of those assessments from the screening visit to the week 8 study visit for the group initially treated with dimebon versus the change from the week 8 study visit to the week 20 study visit for the placebo treated group are also informative.

If desired, trials as described above can be conducted with any of the hydrogenated pyrido [4,3-b] indoles described herein (*e.g*., dimebon), in conjunction with one or more other compounds or therapies useful for treating, preventing and/or delaying the onset and/or development of Alzheimer's disease, to determine the ability of the combination therapy to treat, prevent and/or delay the onset and/or the development of Alzheimer's disease. Standard methods, such as those described in Example 2 for assessing tolerability, pharmacokinetics and pharmacodynamics of a combination therapy can be used. Then a phase II, double-blind randomized controlled trial is performed to determine the efficacy of the combination therapy using standard protocols.

### Example 3. Use of an in vivo model to determine the ability to combination therapies of the invention to treat, prevent and/or delay the onset and/or the development of Alzheimer's disease

*In vivo* models of Alzheimer's disease can also be used to determine the ability of any of the combination therapies described herein to treat, prevent and/or delay the onset and/or the development of Alzheimer's disease in mammals, such as humans. An exemplary animal model of Alzheimer's disease includes transgenic mice over-expressing the 'Swedish' mutant amyloid precursor protein (APP; Tg2576; K670N/M671L; Hsiao et al., 1996, Science, 274:99-102). The phenotype present in these mice has been well-characterized (Holcomb LA et al., 1998, Nat. Med., 4:97-100; Holcomb LA et al., 1999, Behav. Gen., 29:177-185; and McGowan E, 1999, Neurobiol. Dis., 6:231-244). Standard methods can be used to determine whether any of the combination therapies of the invention decrease the amount of Aβ deposits in the brains of these mice (*s*e*e,* for example, WO 2004/032868, published April 22, 2004).

### Example 4. Use of an in vivo model to evaluate the ability of combination therapy with orally-administered dimebon and donepezil to enhance cognition, learning and memory in scopolamine-treated rats.

The two-trial object recognition paradigm developed by Ennaceur and Delacour in the rat was used as a model of episodic memory. Ennaceur, A., and Delacour, J. (1988), Behav. Brain Res. 31:47-59. The paradigm is based on spontaneous exploratory activity of rodents and does not involve rule learning or reinforcement. The object recognition paradigm is sensitive to the effects of ageing and cholinergic dysfunction. *See, e.g.,* Scali, C., et al., (1994), Neurosci. Letts. 170:117-120; and Bartolini, L., et al., (1996), Biochem. Behav. 53:277-283.

Male Sprague-Dawley rats between six and seven weeks old, weighing between 220-300 grams were obtained from Centre d'Elevage (Rue Janvier, B.P. 55, Le Genest-Saint-Isle 53940, France). The animals were housed in groups of 2 to 4 in polypropylene cages (with a floor area of 1032 cm²) under standard conditions: at room temperature (22 ± 2°C), under a 12 hour light/12 hour dark cycle, with food and water provided *ad libitum.* Animals were permitted to acclimate to environmental conditions for at least 5 days before therapy begins, and were numbered on their tails with indelible marker.

The experimental arena was a square wooden box (60 cm × 60 cm × 40 cm) painted dark blue, with 15 cm × 15 cm black squares under a clear plexiglass floor. The arena and objects placed inside the arena were cleaned with water between each trial to eliminate any odor trails left by rats. The arena was placed in a dark room illuminated only by halogen lamps directed towards the ceiling in order to produce a uniformly dim light in the box of approximately 60 lux. The day before testing, animals were allowed to freely explore the experimental arena for three minutes in the presence of two objects (habituation). Animals to be tested were placed in the experimental room at least 30 minutes before testing.

On the day of the experiment, animals were submitted to two trials separated by an interval of 120 minutes. During the first, or acquisition, trial (T₁), rats were placed in the arena, which was prepared with two identical objects. The time required for each animal to complete 15 seconds of object exploration was determined, with a cut-off time of four minutes. Exploration was considered to be directing the nose at a distance less than 2 centimeters ("cm") from the object and/or touching the object. During the second, or testing, trial (T₂), one of the objects presented in the first trial was replaced with an unknown or novel object, while the second, familiar object was left in place. Rats were placed back in the arena for three minutes, and exploration of both objects was determined. Locomotor activity of rats (number of times rats cross grid lines visible under the clear plexiglass floor) was scored for during T₁ and T₂. At the conclusion of the experiments, the rats were sacrificed by an overdose of pentobarbital given intraperitoneally.

The following parameters were measured: (1) time required to achieve 15 seconds of object exploration during T₁; (2) locomotor activity during T₁ (number of crossed lines); (3) time spent in active exploration of the familiar object during T₂ (T_{Familiar}); (4) time spent in active exploration of the novel object during T₂ (T_{Novel}); and (5) locomotor activity during T₂ (number of crossed lines). The difference between time spent in active exploration of the novel object during T₂ and time spent in active exploration of the familiar object during T2 (Δ T_{Novel}-T_{Familiar}) was evaluated. The recognition index [(T_{Novel}-T_{Familiar})/( T_{Novel}+T_{Familiar})] was derived, and the percentage of animals with T_{Novel}-T_{Familiar}> 5 seconds was assessed.

Animals not meeting a minimal level of object exploration were excluded from the study as having naturally low levels of spontaneous exploration. Thus, only rats exploring the objects for at least five seconds (T_{Novel} + T_{Familiar}> 5 seconds) were included in the study.

Animals were randomly assigned to groups of 14. Combination therapy and controls were administered to animals the groups as follows:

| **Group No.** | **Donepezil (route of administration)** | **Scopolamine (route of administration)** | **Dimebon/Vehicle (route of administration)** |
|---|---|---|---|
| 1 | Vehicle (i.p.) | Scopolamine (0.3 mg/kg, i.p.) | Vehicle (p.o.) |
| 2 | Donepezil (0.01 mg/kg, i.p.) | Scopolamine (0.3 mg/kg, i.p.) | Vehicle (p.o.) |
| 3 | Donepezil (0.03 mg/kg, i.p.) | Scopolamine (0.3 mg/kg, i.p.) | Vehicle (p.o.) |
| 4 | Donepezil (1 mg/kg, i.p.) | Scopolamine (0.3 mg/kg, i.p.) | Vehicle (p.o.) |
| 5 | Donepezil (0.01 mg/kg, i.p.) | Scopolamine (0.3 mg/kg, i.p.) | Dimebon (2.5 mg/kg, p.o.) |
| 6 | Donepezil (0.03 mg/kg, i.p.) | Scopolamine (03 mg/kg, i.p.) | Dimebon (2.5 mg/kg, p.o.) |
| 7 | Vehicle (i.p.) | Scopolamine (0.3 mg/kg, i.p.) | Dimebon (5 mg/kg, p.o.) |
| 8 | Vehicle (i.p.) | Scopolamine (0.3 mg/kg, i.p.) | Dimebon (2.5 mg/kg, p.o.) |

| | | | |
|---|---|---|---|
| i.p. = intraperitoneal administration; p.o. *per os* (oral administration); Dimebon = (2,3,4,5-tetrahydro-2,8-dimethyl-5-[2-(6-methyl-3-pyridinyl)ethyl]-1H-pyrido[4,3-b]indole hydrochloride). | | | |

Dimebon (dimebon hydrochloride, M.W. = 392 Daltons) solution was prepared freshly each day at a concentration of 0.25 mg/ml using purified water as vehicle. Donepezil and scopolamine are administered simultaneously in a single solution of saline (5 mL/kg) prepared freshly each day. Scopolamine was purchased from Sigma Chemical Co. (Catalog No.S-1875; St. Quentin Fallavier, France) was dissolved in saline to a concentration of 0.06 mg/mL.

Donepezil or its vehicle and scopolamine were administered intraperitoneally forty minutes before the acquisition trial (T₁). Dimebon or its vehicle were administered by gavage twenty-five minutes before the acquisition trial (T₁), *i.e.,* five minutes after administration of scopolamine. The volume of administration was 5 ml/kg body weight for compounds administered intraperitoneally, and 10 ml/kg for compounds administered orally. The experiments can be repeated substantially as described using any of the hydrogenated pyrido [4,3-b] indoles described herein.

| **Results** | | | |
|---|---|---|---|
| **Group No. (number of animals)*** | **Donepezil (route of administration)** | **Dimebon (route of administration)** | **Memory Performance^{#}** |
| 1 (8) | Vehicle (i.p.) | Vehicle (p.o.) | -0.38 ± 1.10 |
| 2 (8) | Donepezil (0.01 mg/kg, i.p.) | Vehicle (p.o.) | 1.88 ± 0.97 |
| 3 (6) | Donepezil (0.03 mg/kg, i.p.) | Vehicle (p.o.) | 1.17 ± 2.22 |
| 4 (8) | Donepezil (1 mg/kg, i.p.) | Vehicle (p.o.) | 8.8 ± 1.37 |
| 5 (7) | Donepezil (0.01 mg/kg, i.p.) | Dimebon (2.5 mg/kg, p.o.) | -0.86 ± 0.94 |
| 6 (7) | Donepezil (0.03 mg/kg, i.p.) | Dimebon (2.5 mg/kg, p.o.) | 4.71 ± 2.33 |
| 7 (8) | Vehicle (i.p.) | Dimebon (5 mg/kg, p.o.) | 5.00 ± 2.09 |
| 8 (7) | Vehicle (i.p.) | Dimebon (2.5 mg/kg, p.o.) | 2.57 ± 0.95 |
| * **Number of animals per group at the time memory performance data was collected**. | | | |
| # Memory performance expressed as N-F, the difference in time (seconds) spent with the novel object and familiar object. Values are mean ± standard error of the mean. | | | |

In this animal model, maximally effective doses of dimebon and donepezil are 5 mg/kg (oral) and 1 mg/kg (intraperitoneal), respectively. These results demonstrate that administered doses of dimebon (2.5 mg/kg oral) and donepezil (0.03 mg/kg intraperitoneal) below their maximally effective doses have an additive effect when administered in combination. Such doses of dimebon and donepezil are only partially effective when tested individually.

## Claims

1. A compound which is 2,8-dimethyl-5-(2-(6-methyl-3-pyridyl)ethyl)-2,3,4,5-tetrahydro-1H-pyrido[4,3-b]indole, or a pharmaceutically acceptable salt thereof, for use in a method of treating, preventing, slowing the progression or delaying the onset and/or development of Alzheimer's disease in an individual in need thereof, the method comprising administering to the individual an effective amount of (i) said compound; and (ii) donepezil or pharmaceutically acceptable salt thereof.

2. The compound of claim 1 for use as defined in claim 1 in a method of treating Alzheimer's disease.

3. The compound of claim 1 for use as defined in claim 1 or 2, wherein the pharmaceutically acceptable salt of the compound is a pharmaceutically acceptable acid salt, optionally a hydrochloride acid salt.

4. The compound of claim 1 for use as defined in claim 1 or 2, which is 2,8-dimethyl-5-(2-(6-methyl-3-pyridyl)ethyl)-2,3,4,5-tetrahydro- 1H-pyrido[4,3-b]indole dihydrochloride.

5. The compound of any one of claims 1, 3 or 4 for use as defined in any of claims 1 to 4, wherein (ii) comprises donepezil hydrochloride.

6. The compound of any one of claims 1, 3 or 4 for use as defined in any of claims 1 to 5, wherein the method comprises administering to the individual a dose of said compound which is lower than the therapeutic amount required when said compound is used alone.

7. A product comprising: (a) 2,8-dimethyl-5-(2-(6-methyl-3-pyridyl)ethyl)-2,3,4,5-tetrahydro-1H-pyrido[4,3-b]indole, or a pharmaceutically acceptable salt thereof, and (b) donepezil, or a pharmaceutically acceptable salt thereof;
as a combined preparation for simultaneous or sequential use in the treatment, prevention, slowing the progression or delaying the onset and/or development of Alzheimer's disease.

8. A product as defined in claim 7 as a combined preparation for simultaneous or sequential use in the treatment of Alzheimer's disease.

9. A product as defined in claim 7 or 8 wherein (a) comprises a pharmaceutically acceptable acid salt of 2,8-dimethyl-5-(2-(6-methyl-3-pyridyl)ethyl)-2,3,4,5-tetrahydro-1H-pyrido[4,3-b]indole.

10. A product as defined in claim 7 or 8 wherein (a) comprises 2,8-dimethyl-5-(2-(6-methyl-3-pyridyl)ethyl)-2,3,4,5-tetrahydro-1H-pyrido[4,3-b]indole dihydrochloride.

11. A product as defined in any one of claims 7 to 10 in which (b) comprises donepezil hydrochloride.

12. A product as defined in any one of claims 7 to 11 in which (a) and (b) are present in a unit dosage form.

13. A product as defined in claim 12 in which (a) is present in a dose which is lower than the therapeutic amount required when (a) is used alone.

14. A pharmaceutical composition comprising:
(a) 2,8-dimethyl-5-(2-(6-methyl-3-pyridyl)ethyl)-2,3,4,5-tetrahydro-1H-pyrido[4,3-b]indole, or a pharmaceutically acceptable salt thereof;
(b) donepezil, or a pharmaceutically acceptable salt thereof; and
(c) a pharmaceutically acceptable carrier.

15. The pharmaceutical composition of claim 14, wherein (a) comprises a pharmaceutically acceptable acid salt of 2,8-dimethyl-5-(2-(6-methyl-3-pyridyl)ethyl)-2,3,4,5-tetrahydro-1H-pyrido[4,3-b]indole.

16. The pharmaceutical composition of claim 14 wherein (a) comprises 2,8-dimethyl-5-(2-(6-methyl-3-pyridyl)ethyl)-2,3,4,5-tetrahydro-1H-pyrido[4,3-b]indole dihydrochloride.

17. The pharmaceutical composition of claim 14, 15 or 16, wherein (b) comprises donepezil hydrochloride.

18. The pharmaceutical composition of any one of claims 14 to 17 in a unit dosage form.

19. The pharmaceutical composition of claim 18 in which (a) is present in a dose which is lower than the therapeutic amount required when (a) is used alone.

20. Use of a compound which is 2,8-dimethyl-5-(2-(6-methyl-3-pyridyl)ethyl)-2,3,4,5-tetrahydro-1H-pyrido[4,3-b]indole, or a pharmaceutically acceptable salt thereof, in the manufacture of a medicament for treating, preventing, slowing the progression or delaying the onset and/or development of Alzheimer's disease in an individual in need thereof in a method comprising administering to the individual an effective amount of (i) said compound; and (ii) donepezil, or a pharmaceutically acceptable salt thereof.

21. Use according to claim 20 in the manufacture of a medicament for the treatment of Alzheimer's disease.

22. Use according to claim 20 or 21 wherein (i) comprises a pharmaceutically acceptable acid salt of 2,8-dimethyl-5-(2-(6-methyl-3-pyridyl)ethyl)-2,3,4,5-tetrahydro-1H-pyrido[4,3-b]indole.

23. Use according to claim 20 or 21 wherein the (i) comprises 2,8-dimethyl-5-(2-(6-methyl-3-pyridyl)ethyl)-2,3,4,5-tetrahydro-1H-pyrido[4,3-b]indole dihydrochloride.

24. Use according to any one of claims 20 to 23, wherein (ii) comprises donepezil hydrochloride.

25. Use according to any one of claims 20 to 24, wherein (i) comprises a dose of said compound which is lower than the therapeutic amount required when said compound is used alone.

## Patentansprüche

1. Verbindung, welche 2,8-Dimethyl-5-(2-(6-methyl-3-pyridyl)ethyl)-2,3,4,5-tetrahydro-1H-pyrido[4,3-b]indol ist, oder ein pharmazeutisch akzeptables Salz davon, zur Verwendung bei einer Methode zur Behandlung, Verhütung, Verlangsamung des Fortschreitens oder Verzögerung des Auftretens und/oder der Entwicklung der Alzheimer-Krankheit bei einem Individuum, das dessen bedarf, wobei die Methode das Verabreichen an das Individuum einer wirksamen Menge (i) der Verbindung; und (ii) von Donepezil, oder eines pharmazeutisch akzeptablen Salzes davon, umfasst.

2. Verbindung nach Anspruch 1, zur Verwendung wie in Anspruch 1 definiert bei einer Methode zur Behandlung der Alzheimer-Krankheit.

3. Verbindung nach Anspruch 1, zur Verwendung wie in Anspruch 1 oder 2 definiert, wobei das pharmazeutisch akzeptable Salz der Verbindung ein pharmazeutisch akzeptables Säuresalz, optional ein Hydrochlorid-Säuresalz, ist.

4. Verbindung nach Anspruch 1, zur Verwendung wie in Anspruch 1 oder 2 definiert, welche 2,8-Dimethyl-5-(2-(6-methyl-3-pyridyl)ethyl)-2,3,4,5-tetrahydro-1H-pyrido[4,3-b]indol-Dihydrochlorid ist.

5. Verbindung nach jedem der Ansprüche 1, 3 oder 4, zur Verwendung wie in jedem der Ansprüche 1 bis 4 definiert, wobei (ii) Donepezil-Hydrochlorid umfasst.

6. Verbindung nach jedem der Ansprüche 1, 3 oder 4, zur Verwendung wie in jedem der Ansprüche 1 bis 5 definiert, wobei die Methode das Verabreichen an das Individuum einer Dosis der Verbindung umfasst, die geringer als die therapeutisch erforderliche Menge ist, wenn die Verbindung alleine verwendet wird.

7. Produkt, umfassend: (a) 2,8-Dimethyl-5-(2-(6-methyl-3-pyridyl)ethyl)-2,3,4,5-tetrahydro-1H-pyrido[4,3-b]indol, oder ein pharmazeutisch akzeptables Salz davon, und (b) Donepezil, oder ein pharmazeutisch akzeptables Salz davon;
als ein kombiniertes Präparat zur gleichzeitigen oder aufeinanderfolgenden Anwendung in der Behandlung, Verhütung, Verlangsamung des Fortschreitens oder Verzögerung des Auftretens und/oder der Entwicklung der Alzheimer-Krankheit.

8. Produkt, wie in Anspruch 7 definiert, als einem kombinierten Präparat zur gleichzeitigen oder aufeinanderfolgenden Anwendung in der Behandlung der Alzheimer-Krankheit.

9. Produkt, wie in Anspruch 7 oder 8 definiert, wobei (a) ein pharmazeutisch akzeptables Säuresalz von 2,8-Dimethyl-5-(2-(6-methyl-3-pyridyl)ethyl)-2,3,4,5-tetrahydro-1H-pyrido[4,3-b]indol umfasst.

10. Produkt, wie in Anspruch 7 oder 8 definiert, wobei (a) 2,8-Dimethyl-5-(2-(6-methyl-3-pyridyl)ethyl)-2,3,4,5-tetrahydro-1H-pyrido[4,3-b]indol-Dihydrochlorid umfasst.

11. Produkt, wie in jedem der Ansprüche 7 bis 10 definiert, worin (b) Donepezil-Hydrochlorid umfasst.

12. Produkt, wie in jedem der Ansprüche 7 bis 11 definiert, worin (a) und (b) in einer Einheitendosierungsform vorliegen.

13. Produkt, wie in Anspruch 12 definiert, worin (a) in einer Dosierung vorliegt, die geringer ist als die therapeutisch erforderliche Menge, wenn (a) alleine verwendet wird.

14. Pharmazeutische Zusammensetzung, umfassend:
(a) 2,8-Dimethyl-5-(2-(6-methyl-3-pyridyl)ethyl)-2,3,4,5-tetrahydro-1H-pyrido[4,3-b]indol, oder ein pharmazeutisch akzeptables Salz davon;
(b) Donepezil, oder ein pharmazeutisch akzeptables Salz davon; und
(c) einen pharmazeutisch akzeptablen Träger.

15. Pharmazeutische Zusammensetzung nach Anspruch 14, worin (a) ein pharmazeutisch akzeptables Säuresalz von 2,8-Dimethyl-5-(2-(6-methyl-3-pyridyl)ethyl)-2,3,4,5-tetrahydro-1H-pyrido[4,3-b]indol umfasst.

16. Pharmazeutische Zusammensetzung nach Anspruch 14, worin
(a) 2,8-Dimethyl-5-(2-(6-methyl-3-pyridyl)ethyl)-2,3,4,5-tetrahydro-1H-pyrido[4,3-b]indol-Dihydrochlorid umfasst.

17. Pharmazeutische Zusammensetzung nach Anspruch 14, 15 oder 16, worin (b) Donepezil-Hydrochlorid umfasst.

18. Pharmazeutische Zusammensetzung nach jedem der Ansprüche 14 bis 17 in einer Einheitendosierungsform.

19. Pharmazeutische Zusammensetzung nach Anspruch 18, worin (a) in einer Dosierung vorliegt, die geringer ist als die therapeutisch erforderliche Menge, wenn (a) alleine verwendet wird.

20. Verwendung einer Verbindung, welche 2,8-Dimethyl-5-(2-(6-methyl-3-pyridyl)ethyl)-2,3,4,5-tetrahydro-1H-pyrido[4,3-b]indol, oder ein pharmazeutisch akzeptables Salz davon ist, in der Herstellung eines Medikaments zur Behandlung, Verhütung, Verlangsamung des Fortschreitens oder Verzögerung des Auftretens und/oder der Entwicklung der Alzheimer-Krankheit bei einem Individuum, das dessen bedarf, bei einer Methode, umfassend das Verabreichen an das Individuum einer wirksamen Menge (i) der Verbindung; und (ii) an Donepezil, oder eines pharmazeutisch akzeptablen Salzes davon.

21. Verwendung nach Anspruch 20 in der Herstellung eines Medikaments zur Behandlung der Alzheimer-Krankheit.

22. Verwendung nach Anspruch 20 oder 21, wobei (i) ein pharmazeutisch akzeptables Säuresalz von 2,8-Dimethyl-5-(2-(6-methyl-3-pyridyl)ethyl)-2,3,4,5-tetrahydro-1H-pyrido[4,3-b]indol umfasst.

23. Verwendung nach Anspruch 20 oder 21, wobei (i) 2,8-Dimethyl-5-(2-(6-methyl-3-pyridyl)ethyl)-2,3,4,5-tetrahydro-1H-pyrido[4,3-b]indol-Dihydrochlorid umfasst.

24. Verwendung nach jedem der Ansprüche 20 bis 23, wobei (ii) Donepezil-Hydrochlorid umfasst.

25. Verwendung nach jedem der Ansprüche 20 bis 24, wobei (i) eine Dosis der Verbindung umfasst, die geringer ist als die therapeutisch erforderliche Menge, wenn (a) alleine verwendet wird.

## Revendications

1. Composé qui est le 2,8-diméthyl-5-[2-(6-méthyl-pyrid-3-yl)-éthyl]-2,3,4,5-tétrahydro-1H-pyrido[4,3-b]indole, ou un sel pharmacologiquement admissible de ce composé, pour son utilisation dans un procédé permettant, chez un individu qui en a besoin, de traiter ou de prévenir la maladie d'Alzheimer, d'en ralentir la progression ou d'en retarder le déclenchement et/ou le développement, lequel procédé comporte le fait d'administrer audit individu, en quantité efficace,
i) dudit composé,
ii) et du donépézil ou un sel pharmacologiquement admissible de ce composé.

2. Composé conforme à la revendication 1, pour son utilisation, définie dans la revendication 1, dans un procédé de traitement de la maladie d'Alzheimer.

3. Composé conforme à la revendication 1, pour son utilisation définie dans la revendication 1 ou 2, le sel pharmacologiquement admissible de ce composé étant un sel d'acide pharmacologiquement admissible, en option un sel d'acide de type chlorhydrate.

4. Composé conforme à la revendication 1, pour son utilisation définie dans la revendication 1 ou 2, qui est le dichlorhydrate de 2,8-diméthyl-5-[2-(6-méthyl-pyrid-3-yl)-éthyl]-2,3,4,5-tétrahydro-1H-pyrido[4,3-b]indole.

5. Composé conforme à l'une des revendications 1, 3 et 4, pour son utilisation définie dans l'une des revendications 1 à 4, le composé (ii) comprenant du chlorhydrate de donépézil.

6. Composé conforme à l'une des revendications 1, 3 et 4, pour son utilisation définie dans l'une des revendications 1 à 5, ledit procédé comprenant le fait d'administrer audit individu ledit composé en une dose plus faible que la quantité qui est requise d'un point de vue thérapeutique quand ledit composé est utilisé seul.

7. Produit comprenant :
a) du 2,8-diméthyl-5-[2-(6-méthyl-pyrid-3-yl)-éthyl]-2,3,4,5-tétrahydro-1H-pyrido[4,3-b]indole, ou un sel pharmacologiquement admissible de ce composé,
b) et du donépézil ou un sel pharmacologiquement admissible de ce composé,
sous forme de préparation combinée pour utilisation en mode simultané ou consécutif dans le fait de traiter ou de prévenir la maladie d'Alzheimer, d'en ralentir la progression ou d'en retarder le déclenchement et/ou le développement.

8. Produit conforme à la revendication 7, sous forme de préparation combinée pour utilisation en mode simultané ou consécutif dans le traitement de la maladie d'Alzheimer.

9. Produit conforme à la revendication 7 ou 8, dans lequel le composant (a) comprend un sel d'acide pharmacologiquement admissible du 2,8-diméthyl-5-[2-(6-méthyl-pyrid-3-yl)-éthyl]-2,3,4,5-tétrahydro-1H-pyrido[4,3-b]indole.

10. Produit conforme à la revendication 7 ou 8, dans lequel le composant (a) comprend du dichlorhydrate de 2,8-diméthyl-5-[2-(6-méthyl-pyrid-3-yl)-éthyl]-2,3,4,5-tétrahydro-1H-pyrido[4,3-b]indole.

11. Produit conforme à l'une des revendications 7 à 10, dans lequel le composant (b) comprend du chlorhydrate de donépézil.

12. Produit conforme à l'une des revendications 7 à 11, dans lequel les composants (a) et (b) se trouvent sous une présentation pour dose unitaire.

13. Produit conforme à la revendication 12, dans lequel le composant (a) se trouve présent en une dose plus faible que la quantité qui est requise d'un point de vue thérapeutique quand ce composant (a) est utilisé seul.

14. Composition pharmaceutique comprenant :
a) du 2,8-diméthyl-5-[2-(6-méthyl-pyrid-3-yl)-éthyl]-2,3,4,5-tétrahydro-1H-pyrido[4,3-b]indole, ou un sel pharmacologiquement admissible de ce composé,
b) du donépézil ou un sel pharmacologiquement admissible de ce composé,
c) et un véhicule pharmacologiquement admissible.

15. Composition pharmaceutique conforme à la revendication 14, dans laquelle le composant (a) comprend un sel d'acide pharmacologiquement admissible du 2,8-diméthyl-5-[2-(6-méthyl-pyrid-3-yl)-éthyl]-2,3,4,5-tétrahydro-1H-pyrido[4,3-b]indole.

16. Composition pharmaceutique conforme à la revendication 14, dans laquelle le composant (a) comprend du dichlorhydrate de 2,8-diméthyl-5-[2-(6-méthyl-pyrid-3-yl)-éthyl]-2,3,4,5-tétrahydro-1H-pyrido[4,3-b]indole.

17. Composition pharmaceutique conforme à la revendication 14, 15 ou 16, dans lequel le composant (b) comprend du chlorhydrate de donépézil.

18. Composition pharmaceutique conforme à l'une des revendications 14 à 17, sous une présentation pour dose unitaire.

19. Composition pharmaceutique conforme à la revendication 18, dans laquelle le composant (a) se trouve présent en une dose plus faible que la quantité qui est requise d'un point de vue thérapeutique quand ce composant (a) est utilisé seul.

20. Utilisation d'un composé qui est le 2,8-diméthyl-5-[2-(6-méthyl-pyrid-3-yl)-éthyl]-2,3,4,5-tétrahydro-1H-pyrido[4,3-b]indole, ou un sel pharmacologiquement admissible de ce composé, dans la fabrication d'un médicament conçu pour traiter ou prévenir la maladie d'Alzheimer, en ralentir la progression ou en retarder le déclenchement et/ou le développement, chez un individu qui en a besoin, dans un procédé comportant le fait d'administrer audit individu, en quantité efficace,
i) dudit composé,
ii) et du donépézil ou un sel pharmacologiquement admissible de ce composé.

21. Utilisation, conforme à la revendication 20, dans la fabrication d'un médicament conçu pour le traitement de la maladie d'Alzheimer.

22. Utilisation conforme à la revendication 20 ou 21, pour laquelle le composant (i) comprend un sel d'acide pharmacologiquement admissible du 2,8-diméthyl-5-[2-(6-méthyl-pyrid-3-yl)-éthyl]-2,3,4,5-tétrahydro-1H-pyrido[4,3-b]indole.

23. Utilisation conforme à la revendication 20 ou 21, pour laquelle le composant (i) comprend du dichlorhydrate de 2,8-diméthyl-5-[2-(6-méthyl-pyrid-3-yl)-éthyl]-2,3,4,5-tétrahydro-1H-pyrido-[4,3-b]indole.

24. Utilisation conforme à l'une des revendications 20 à 23, pour laquelle le composant (ii) comprend du chlorhydrate de donépézil.

25. Utilisation conforme à l'une des revendications 20 à 24, pour laquelle le composant (i) comprend dudit composé en une dose plus faible que la quantité qui est requise d'un point de vue thérapeutique quand ledit composé est utilisé seul.
